# EUROPEAN PATENT APPLICATION

(11) **EP 1 736 540 A1**
(43) Date of publication of application: **27.12.2006**
(21) Application number: 05719990.3
(22) Date of filing: 04.03.2005
(51) Int. Cl.: C12N 15/09, C12P 21/02

(54) **PROCESS FOR PRODUCING DIPEPTIDES**

(30) Priority: 29.03.2004 JP 2004093953
(71) Applicant: KYOWA HAKKO KOGYO CO., LTD., Chiyoda-ku, Tokyo 100-8185 (JP)
(72) Inventor: KINO, Kuniki, Chiba-shi, Chiba 262-0026 (JP); SATO, Masaru, Kanagawa 228-0015 (JP)
(74) Representative: Vossius & Partner
(86) International application number: PCT/JP2005/003718
(87) International publication number: WO 2005/093056

(57) **Abstract**

The present invention provides a process for producing a dipeptide other than D-alanyl-D-alanine and D-alanyl-D-serine using, as an enzyme source, a culture of a microorganism having the ability to produce a protein having D-alanine-D-alanine ligase activity or a treated matter of the culture, and a process for producing a dipeptide comprising D-amino acid using, as enzyme sources, a culture of a microorganism having the ability to produce the D-amino acid or a treated matter of the culture and a culture of a microorganism having the ability to produce a protein having D-alanine-D-alanine ligase activity or a treated matter of the culture.

SEQUENCE LISTING
<110> KYOWA HAKKO KOGYO CO., LTD.

<120> Process for producing dipeptides

<130> 1664

<150> JP2004-93953
<151> 2004-03-29

<160> 30

<170> PatentIn Ver. 2.1

<210> 1
<211> 306
<212> PRT
<213> Escherichia coli K-12

<400> 1


<210> 2
<211> 364
<212> PRT
<213> Escherichia coli K-12

<400> 2
Met Glu Lys Leu Arg Val Gly Ile Val Phe Gly Gly Lys Ser Ala Glu
  1               5                  10                  15

His Glu Val Ser Leu Gln Ser Ala Lys Asn Ile Val Asp Ala Ile Asp
             20                  25                  30

Lys Ser Arg Phe Asp Val Val Leu Leu Gly Ile Asp Lys Gln Gly Gln
         35                  40                  45

Trp His Val Ser Asp Ala Ser Asn Tyr Leu Leu Asn Ala Asp Asp Pro
     50                  55                  60

Ala His Ile Ala Leu Arg Pro Ser Ala Thr Ser Leu Ala Gln Val Pro
 65                  70                  75                  80

Gly Lys His Glu His Gln Leu Ile Asp Ala Gln Asn Gly Gln Pro Leu
                 85                  90                  95

Pro Thr Val Asp Val Ile Phe Pro Ile Val His Gly Thr Leu Gly Glu
            100                 105                 110

Asp Gly Ser Leu Gln Gly Met Leu Arg Val Ala Asn Leu Pro Phe Val
        115                 120                 125

Gly Ser Asp Val Leu Ala Ser Ala Ala Cys Met Asp Lys Asp Val Thr
    130                 135                 140

Lys Arg Leu Leu Arg Asp Ala Gly Leu Asn Ile Ala Pro Phe Ile Thr
145                 150                 155                 160

Leu Thr Arg Ala Asn Arg His Asn Ile Ser Phe Ala Glu Val Glu Ser
                165                 170                 175

Lys Leu Gly Leu Pro Leu Phe Val Lys Pro Ala Asn Gln Gly Ser Ser
            180                 185                 190

Val Gly Val Ser Lys Val Thr Ser Glu Glu Gln Tyr Ala Ile Ala Val
        195                 200                 205

Asp Leu Ala Phe Glu Phe Asp His Lys Val Ile Val Glu Gln Gly Ile
    210                 215                 220

Lys Gly Arg Glu Ile Glu Cys Ala Val Leu Gly Asn Asp Asn Pro Gln
225                 230                 235                 240

Ala Ser Thr Cys Gly Glu Ile Val Leu Thr Ser Asp Phe Tyr Ala Tyr
                245                 250                 255

Asp Thr Lys Tyr Ile Asp Glu Asp Gly Ala Lys Val Val Val Pro Ala

260                 265                 270

Ala Ile Ala Pro Glu Ile Asn Asp Lys Ile Arg Ala Ile Ala Val Gln
        275                 280                 285

Ala Tyr Gln Thr Leu Gly Cys Ala Gly Met Ala Arg Val Asp Val Phe
    290                 295                 300

Leu Thr Pro Glu Asn Glu Val Val Ile Asn Glu Ile Asn Thr Leu Pro
305                 310                 315                 320

Gly Phe Thr Asn Ile Ser Met Tyr Pro Lys Leu Trp Gln Ala Ser Gly
                325                 330                 335

Leu Gly Tyr Thr Asp Leu Ile Thr Arg Leu Ile Glu Leu Ala Leu Glu
            340                 345                 350

Arg His Ala Ala Asp Asn Ala Leu Lys Thr Thr Met
        355                 360


<210> 3
<211> 354
<212> PRT
<213> Oceanobacillus oheyensis HTE831

<400> 3
Met Arg Val Gly Leu Leu Phe Gly Gly Cys Ser Gly Glu His Glu Val
1               5                   10                  15

Ser Ile Lys Ser Ala Gln Ala Ile Ala Lys Ala Leu Ala Ser Asp Asp
            20                  25                  30

Asn Gln Thr Lys Tyr Gln Val Ser Pro Phe Tyr Ile Gln Lys Asn Gly
        35                  40                  45

Val Trp Leu Gly Pro Asp Val Ser Gln Gln Val Leu Asp Gln Gly Val
    50                  55                  60

Pro Trp Gly Asp Gln Pro Val Thr Ala Gly Gln Arg Trp Gln Phe Pro
65                  70                  75                  80

Pro Glu Ala Ala Arg Met Glu Val Trp Phe Pro Ile Leu His Gly Pro
                85                  90                  95

Asn Gly Glu Asp Gly Thr Val Gln Gly Leu Phe Ser Leu Met Gln Val
            100                 105                 110

Pro Tyr Val Gly Ser Gly Val Leu Gly Ser Cys Val Gly Met Asp Lys
        115                 120                 125

Leu Ala Met Lys Met Val Phe Glu Arg Ala Gly Leu Pro Gln Val Asn
    130                 135                 140

Tyr Met Gly Val Glu Arg Gly Glu Ile Trp Ser Asn Pro Cys Val Phe
145                 150                 155                 160

Pro Ala Leu Cys Glu Lys Ile Glu Ala Gln Val Gly Tyr Pro Cys Phe
                165                 170                 175

Val Lys Pro Ala Asn Leu Gly Ser Ser Val Gly Ile Ala Lys Val Arg
            180                 185                 190

Asn Arg Ser Glu Leu Glu Ala Ala Leu Asp Asn Ala Ala Ser Tyr Asp
        195                 200                 205

Arg Arg Ile Ile Val Glu Ala Gly Leu Thr Asp Ile Arg Glu Val Glu
    210                 215                 220

Cys Ala Val Leu Gly Asn Glu Asn Pro Gln Ala Ser Val Val Gly Glu
225                 230                 235                 240

Ile Thr Tyr Asp Ser Asp Phe Tyr Asp Tyr Glu Thr Lys Tyr Thr Asp
                245                 250                 255

Gly Arg Ser Gln Met His Ile Pro Ala Asn Leu Pro Lys Ala Val Val
            260                 265                 270

Asn Gln Ile Gln Thr Met Ala Ile Gln Ala Phe Lys Ala Val Asp Ala
        275                 280                 285

Ala Gly Leu Gly Arg Leu Asp Phe Phe Tyr Gln Pro Thr Thr Gly Gln
    290                 295                 300

Ile Val Ile Asn Glu Ile Asn Thr Leu Pro Gly Phe Thr Ala Phe Ser
305                 310                 315                 320

Met Tyr Pro Glu Leu Trp Arg Ala Ser Gly Val Glu Phe Pro Ser Leu
                325                 330                 335

Val Asp Arg Leu Leu Gln Leu Ala Leu Asp Tyr His Gly Arg Pro Gly
            340                 345                 350

His Gln


<210> 4
<211> 366
<212> PRT
<213> Synechocystis sp. PPC6806

<400> 4
Met Lys Lys Lys Val Gly Ile Val Phe Gly Gly Lys Ser Ala Glu His
  1               5                  10                  15

Glu Val Ser Leu Gln Ser Ala Lys Asn Ile Val Asp Ala Ile Asp Ser
             20                  25                  30

Asp Lys Tyr Asp Val Tyr Leu Leu Gly Ile Asp Lys Gln Gly Asn Trp
         35                  40                  45

His Val Asn Asp Arg Ser His Tyr Leu Ile Asn Glu Glu Asp Pro Lys
     50                  55                  60

Leu Ile Ser Leu Asn Lys Thr Asn Glu Gly Ile Ala Val Ile Pro Gly
 65                  70                  75                  80

Ala Glu Lys Asn Gln Ile Val Thr Ser Asn Ser Thr Lys Ser Leu Asp
                 85                  90                  95

Gln Leu Asp Val Ile Phe Pro Ile Val His Gly Thr Leu Gly Glu Asp
            100                 105                 110

Gly Ser Leu Gln Gly Met Leu Arg Met Ala Asn Ile Pro Phe Val Gly
        115                 120                 125

Ser Ser Val Leu Gly Ser Ala Ile Ser Met Asp Lys Asp Ile Ala Lys
    130                 135                 140

Arg Leu Leu Lys Asp Ala Gly Leu Lys Val Ala Lys Gly Tyr Ala Tyr
145                 150                 155                 160

Arg Ser Val Asp Lys Glu Ser Ile His Phe Asp Ser Leu Lys Glu Glu
                165                 170                 175

Leu Gly Leu Pro Met Phe Ile Lys Pro Ala Asn Gln Gly Ser Ser Val

180                 185                 190

Gly Val His Lys Val Glu Asn Glu Glu Gln Phe Tyr Ser Ala Ile Lys
        195                 200                 205

Asp Ala Phe Gln Phe Asp His Lys Leu Leu Val Glu Glu Ala Ile Val
    210                 215                 220

Gly Arg Glu Ile Glu Cys Ala Val Leu Gly Asn Glu Lys Pro Ala Ala
225                 230                 235                 240

Ser Val Pro Gly Glu Ile Leu Pro Thr Gly Asp Phe Tyr Ser Tyr Glu
                245                 250                 255

Ala Lys Tyr Ile Asp Glu Thr Gly Ala Val Leu Gln Ile Pro Ala Lys
            260                 265                 270

Leu Glu Glu Pro Val Val Asp Ser Ile Gln Asp Ile Ala Leu Gln Ala
        275                 280                 285

Phe Lys Ala Leu Asn Cys Glu Gly Leu Ala Arg Val Asp Val Phe Leu
    290                 295                 300

Thr Asp Asp Asn Glu Ile Val Ile Asn Glu Ile Asn Thr Leu Pro Gly
305                 310                 315                 320

Phe Thr Lys Ile Ser Met Tyr Pro Lys Leu Trp Glu Glu Ser Gly Val
                325                 330                 335

Ser Tyr Arg Glu Leu Ile Glu Asn Leu Ile Glu Leu Ala Ile Glu Arg
            340                 345                 350

Asn Gln Arg Asp Gln Gln Leu Lys Ser Ser Arg Ser Asp Gly
        355                 360                 365


<210> 5
<211> 303
<212> PRT
<213> Thermotoga maritima ATCC 43589

<400> 5
Met Arg Val Ala Leu Leu Met Gly Gly Val Ser Arg Glu Arg Glu Ile
1               5                   10                  15

Ser Leu Arg Ser Gly Glu Arg Val Lys Lys Ala Leu Glu Lys Leu Gly
            20                  25                  30

Tyr Glu His Thr Val Phe Asp Val Arg Glu Asp Phe Leu Lys Lys Val
        35                  40                  45

Asp Gln Leu Lys Ser Phe Asp Val Val Phe Asn Val Leu His Gly Thr
    50                  55                  60

Phe Gly Glu Asp Gly Thr Leu Gln Ala Ile Leu Asp Phe Leu Gly Ile
65                  70                  75                  80

Arg Tyr Thr Gly Ser Asp Ala Phe Ser Ser Met Ile Cys Phe Asp Lys
                85                  90                  95

Leu Val Thr Tyr Arg Phe Leu Lys Gly Thr Val Glu Ile Pro Asp Phe
            100                 105                 110

Val Glu Ile Lys Glu Phe Met Lys Thr Ser Pro Leu Gly Tyr Pro Cys
        115                 120                 125

Val Val Lys Pro Arg Arg Glu Gly Ser Ser Ile Gly Val Phe Val Cys
    130                 135                 140

Glu Ser Asp Glu Glu Phe Gln His Ala Leu Lys Glu Asp Leu Pro Arg
145                 150                 155                 160

Tyr Gly Ser Val Ile Val Gln Lys Tyr Ile Pro Gly Arg Glu Met Thr
                165                 170                 175

Val Ser Ile Leu Glu Thr Glu Lys Gly Phe Glu Ile Leu Pro Val Leu
            180                 185                 190

Glu Leu Arg Pro Lys Arg Arg Phe Tyr Asp Tyr Val Ala Lys Tyr Thr
        195                 200                 205

Lys Gly Glu Thr Glu Phe Ile Leu Pro Ala Pro Leu Asn Pro Ser Glu
    210                 215                 220

Glu Arg Leu Val Lys Glu Met Ala Leu Lys Ala Phe Val Glu Ala Gly
225                 230                 235                 240

Cys Arg Gly Phe Gly Arg Val Asp Gly Ile Phe Ser Asp Gly Arg Phe
                245                 250                 255

Tyr Phe Leu Glu Ile Asn Thr Val Pro Gly Leu Thr Glu Thr Ser Asp
            260                 265                 270

Leu Pro Ala Ser Ala Lys Ala Gly Gly Ile Glu Phe Glu Glu Leu Val
        275                 280                 285

Glu Ile Ile Leu Lys Ser Ala Phe Leu Lys Glu Gly Val Arg Val
    290                 295                 300

<210> 6
<211> 918
<212> DNA
<213> Escherichia coli K-12

<400> 6
atg act gat aaa atc gcg gtc ctg ttg ggt ggg acc tcc gct gag cgg     48
gaa gtt tct ctg aat tct ggc gca gcg gtg tta gcc gga ctg cgt gaa     96
ggc ggt att gac gcg tat cct gtc gac ccg aaa gaa gtc gac gtg acg     144
caa ctg aag tcg atg ggc ttt cag aaa gtg ttt atc gcg cta cac ggt     192
cgc ggc ggt gaa gat ggt acg ctg cag ggg atg ctc gag ctg atg ggc     240
ttg cct tat acc gga agc gga gtg atg gca tct gcg ctt tca atg gat     288
aaa cta cgc agc aaa ctt cta tgg caa ggt gcc ggt tta ccg gtc gcg     336
ccg tgg gta gcg tta acc cgc gca gag ttt gaa aaa ggc ctg agc gat     384
aag cag tta gca gaa att tct gct ctg ggt ttg ccg gtt atc gtt aag      432
ccg agc cgc gaa ggt tcc agt gtg gga atg tca aaa gta gta gca gaa      480
aat gct cta caa gat gca tta aga ttg gca ttt cag cac gat gaa gaa      528
gta ttg att gaa aaa tgg cta agt ggg ccg gag ttc acg gtt gcg ata      576
ctc ggt gaa gaa att tta ccg tca ata cgt att caa ccg tcc gga acc      624
ttc tat gat tat gag gcg aag tat ctc tct gat gag aca cag tat ttc      672
tgc ccc gca ggt ctg gaa gcg tca caa gag gcc aat ttg cag gca tta      720
gtg ctg aaa gca tgg acg acg tta ggt tgc aaa gga tgg gga cgt att      768
gac gtt atg ctg gac agc gat gga cag ttt tat ctg ctg gaa gcc aat      816
acc tca ccg ggt atg acc agc cac agc ctg gtg ccg atg gcg gca cgt      864
cag gca ggt atg agc ttc tcg cag ttg gta gta cga att ctg gaa ctg      912
gcg gac                                                              918

<210> 7
<211> 1092
<212> DNA
<213> Escherichia coli K-12

<400> 7
atg gaa aaa ctg cgg gta gga atc gtt ttt ggt ggt aaa tca gcg gaa      48
Met Glu Lys Leu Arg Val Gly Ile Val Phe Gly Gly Lys Ser Ala Glu
  1               5                  10                  15

cat gaa gtg tct ctg caa tcg gca aaa aac att gtc gat gcc att gat      96
His Glu Val Ser Leu Gln Ser Ala Lys Asn Ile Val Asp Ala Ile Asp
                20                  25                  30

aaa agt cgc ttc gac gtt gtg ctg ctg ggc att gat aaa caa ggg caa      144
Lys Ser Arg Phe Asp Val Val Leu Leu Gly Ile Asp Lys Gln Gly Gln
            35                  40                  45

tgg cac gtc agc gat gcc agc aat tat ctg cta aat gca gac gat cct     192
Trp His Val Ser Asp Ala Ser Asn Tyr Leu Leu Asn Ala Asp Asp Pro
    50                  55                  60

gcc cat att gcg ttg cgc cct tcg gcg acc agc ctt gcg cag gtg cca     240
Ala His Ile Ala Leu Arg Pro Ser Ala Thr Ser Leu Ala Gln Val Pro
65                  70                  75                  80

ggt aaa cat gag cat cag ctt atc gac gcg caa aac ggt cag ccg ttg     288
Gly Lys His Glu His Gln Leu Ile Asp Ala Gln Asn Gly Gln Pro Leu
                85                  90                  95

ccg acg gtg gat gtc att ttc ccg att gtc cac ggt acg ctg ggc gaa     336
Pro Thr Val Asp Val Ile Phe Pro Ile Val His Gly Thr Leu Gly Glu
            100                 105                 110

gat ggt tcc ttg cag gga atg ctg cgg gtc gcc aat tta ccg ttt gta     384
Asp Gly Ser Leu Gln Gly Met Leu Arg Val Ala Asn Leu Pro Phe Val
        115                 120                 125

ggt tct gat gtt ctg gct tca gca gcc tgt atg gac aaa gat gtc acc     432
Gly Ser Asp Val Leu Ala Ser Ala Ala Cys Met Asp Lys Asp Val Thr
    130                 135                 140

aaa cgt ctg cta cgc gat gcc ggg ctg aac att gcg cca ttt att acc     480
Lys Arg Leu Leu Arg Asp Ala Gly Leu Asn Ile Ala Pro Phe Ile Thr
145                 150                 155                 160

ctg acg cgc gcc aat cgt cac aac att agt ttt gcc gaa gtg gag tct     528
Leu Thr Arg Ala Asn Arg His Asn Ile Ser Phe Ala Glu Val Glu Ser
                165                 170                 175

aaa ctg ggg tta cca ctg ttt gta aaa ccg gct aat cag ggc tct tct     576
Lys Leu Gly Leu Pro Leu Phe Val Lys Pro Ala Asn Gln Gly Ser Ser
            180                 185                 190

gtt ggt gtc agc aaa gta acc agt gaa gaa cag tac gca att gcc gtc     624
Val Gly Val Ser Lys Val Thr Ser Glu Glu Gln Tyr Ala Ile Ala Val
        195                 200                 205

gat ctg gcg ttc gag ttc gat cat aaa gtg atc gtt gag caa ggg atc     672
Asp Leu Ala Phe Glu Phe Asp His Lys Val Ile Val Glu Gln Gly Ile
    210                 215                 220

aaa ggt cgt gag atc gaa tgc gca gtt ctg ggc aac gac aat ccg caa     720
Lys Gly Arg Glu Ile Glu Cys Ala Val Leu Gly Asn Asp Asn Pro Gln
225                 230                 235                 240

gcc agc acc tgt gga gag atc gta ctc acc agc gat ttc tat gcc tac     768
Ala Ser Thr Cys Gly Glu Ile Val Leu Thr Ser Asp Phe Tyr Ala Tyr
                245                 250                 255

gac acc aag tac att gac gaa gat ggc gcg aaa gtg gta gtt ccg gca     816
Asp Thr Lys Tyr Ile Asp Glu Asp Gly Ala Lys Val Val Val Pro Ala
            260                 265                 270

gcc att gcg cca gaa atc aac gat aag atc cgg gcg att gcc gtt cag     864
Ala Ile Ala Pro Glu Ile Asn Asp Lys Ile Arg Ala Ile Ala Val Gln
        275                 280                 285

gct tat caa acg ttg gga tgc gca ggc atg gcg cgt gta gac gtg ttt     912
Ala Tyr Gln Thr Leu Gly Cys Ala Gly Met Ala Arg Val Asp Val Phe
    290                 295                 300

tta acc cca gag aac gaa gtg gtg atc aac gag atc aac acc ctg cct     960
Leu Thr Pro Glu Asn Glu Val Val Ile Asn Glu Ile Asn Thr Leu Pro
305                 310                 315                 320

ggc ttc act aat atc agt atg tat ccg aag ctg tgg caa gcc agc ggt 1008
Gly Phe Thr Asn Ile Ser Met Tyr Pro Lys Leu Trp Gln Ala Ser Gly
                325                 330                 335

ctg ggt tac acc gat ctg atc aca cgt ttg att gaa ctg gcg ctg gag 1056
Leu Gly Tyr Thr Asp Leu Ile Thr Arg Leu Ile Glu Leu Ala Leu Glu
            340                 345                 350

cgt cac gct gcg gat aac gca ttg aaa acc aca atg 1092
Arg His Ala Ala Asp Asn Ala Leu Lys Thr Thr Met
        355                 360

<210> 8
<211> 1062
<212> DNA
<213> Oceanobacillus oheyensis HTE831

<400> 8
atg cgt gtg ggg ctg tta ttt ggt ggg tgt tct ggg gag cat gaa gtc 48
Met Arg Val Gly Leu Leu Phe Gly Gly Cys Ser Gly Glu His Glu Val
1               5                   10                  15

tcg atc aaa tcc gcc cag gcg atc gcc aaa gca ttg gcc agc gac gat 96
Ser Ile Lys Ser Ala Gln Ala Ile Ala Lys Ala Leu Ala Ser Asp Asp
            20                  25                  30

aac caa acc aaa tat caa gta agc cct ttt tat att caa aaa aat ggc 144
Asn Gln Thr Lys Tyr Gln Val Ser Pro Phe Tyr Ile Gln Lys Asn Gly
        35                  40                  45

gtt tgg ctg ggg cca gac gtt tct caa cag gtt ttg gac cag gga gtt 192
Val Trp Leu Gly Pro Asp Val Ser Gln Gln Val Leu Asp Gln Gly Val
    50                  55                  60

ccc tgg ggg gat cag ccc gtt acg gcc gga caa cgg tgg cag ttt ccc 240
Pro Trp Gly Asp Gln Pro Val Thr Ala Gly Gln Arg Trp Gln Phe Pro
65                  70                  75                  80

ccc gaa gcg gcc cga atg gag gtg tgg ttt ccc att ttg cac ggc ccc 288
Pro Glu Ala Ala Arg Met Glu Val Trp Phe Pro Ile Leu His Gly Pro
                85                  90                  95

aac ggt gaa gat ggc aca gtg cag ggg tta ttt agt ttg atg caa gtg 336
Asn Gly Glu Asp Gly Thr Val Gln Gly Leu Phe Ser Leu Met Gln Val
            100                 105                 110

ccc tat gtg ggc agt ggc gtg ctg ggt tcc tgt gtg ggc atg gat aaa 384
Pro Tyr Val Gly Ser Gly Val Leu Gly Ser Cys Val Gly Met Asp Lys
        115                 120                 125

ttg gcg atg aaa atg gtc ttt gaa cgg gca ggg ttg ccc cag gtc aat 432
Leu Ala Met Lys Met Val Phe Glu Arg Ala Gly Leu Pro Gln Val Asn
    130                 135                 140

tac atg ggg gta gaa cgg ggg gaa att tgg tct aat ccc tgt gtt ttt 480
Tyr Met Gly Val Glu Arg Gly Glu Ile Trp Ser Asn Pro Cys Val Phe
145                 150                 155                 160

ccg gct ttg tgc gaa aaa att gaa gcc cag gtg ggc tat ccc tgt ttc 528
Pro Ala Leu Cys Glu Lys Ile Glu Ala Gln Val Gly Tyr Pro Cys Phe
                165                 170                 175

gtt aaa ccg gcc aac tta ggc tcc tcc gtg ggc att gcc aaa gtg cgg 576
Val Lys Pro Ala Asn Leu Gly Ser Ser Val Gly Ile Ala Lys Val Arg
            180                 185                 190

aac cgt agt gaa ttg gaa gca gcc cta gac aac gcc gcc agt tac gat     624
Asn Arg Ser Glu Leu Glu Ala Ala Leu Asp Asn Ala Ala Ser Tyr Asp
        195                 200                 205

cgc cgc atc att gtc gaa gcg ggt tta acg gat att agg gaa gtg gaa     672
Arg Arg Ile Ile Val Glu Ala Gly Leu Thr Asp Ile Arg Glu Val Glu
    210                 215                 220

tgt gcg gtg ctg ggc aac gaa aat ccc caa gct tcc gtg gtg ggg gaa     720
Cys Ala Val Leu Gly Asn Glu Asn Pro Gln Ala Ser Val Val Gly Glu
225                 230                 235                 240

atc acc tac gac agc gac ttt tac gat tac gaa acc aaa tac acc gat     768
Ile Thr Tyr Asp Ser Asp Phe Tyr Asp Tyr Glu Thr Lys Tyr Thr Asp
                245                 250                 255

ggc cgc tcc caa atg cat atc ccc gcc aac tta cct aaa gct gtg gtg     816
Gly Arg Ser Gln Met His Ile Pro Ala Asn Leu Pro Lys Ala Val Val
            260                 265                 270

aac cag atc cag acc atg gcc att caa gct ttt aaa gct gtg gat gcg     864
Asn Gln Ile Gln Thr Met Ala Ile Gln Ala Phe Lys Ala Val Asp Ala
        275                 280                 285

gcg ggc tta gga cga ttg gac ttt ttc tat caa ccg act acg ggg cag     912
Ala Gly Leu Gly Arg Leu Asp Phe Phe Tyr Gln Pro Thr Thr Gly Gln
    290                 295                 300

att gtc atc aac gaa att aac acc ctg cct ggt ttc acc gcc ttt agt     960
Ile Val Ile Asn Glu Ile Asn Thr Leu Pro Gly Phe Thr Ala Phe Ser
305                 310                 315                 320

atg tat ccc gaa ctg tgg cga gct tct ggg gta gaa ttt cct agc tta     1008
Met Tyr Pro Glu Leu Trp Arg Ala Ser Gly Val Glu Phe Pro Ser Leu
                325                 330                 335

gtg gat cga tta tta cag ttg gca ttg gac tac cat ggc cgt ccc ggt     1056
Val Asp Arg Leu Leu Gln Leu Ala Leu Asp Tyr His Gly Arg Pro Gly
            340                 345                 350

cac caa                                                             1062
His Gln

<210> 9
<211> 1098
<212> DNA
<213> Synechocystis sp. PPC6806

<400> 9
atg aag aaa aaa gtt ggt att gta ttt gga ggt aag tca gca gaa cat     48
Met Lys Lys Lys Val Gly Ile Val Phe Gly Gly Lys Ser Ala Glu His
1               5                   10                  15

gag gtt tca ctg cag tct gct aaa aac att gta gat gca atc gac tca     96
Glu Val Ser Leu Gln Ser Ala Lys Asn Ile Val Asp Ala Ile Asp Ser
            20                  25                  30

gat aaa tac gac gta tac tta tta gga att gat aag caa ggg aat tgg     144
Asp Lys Tyr Asp Val Tyr Leu Leu Gly Ile Asp Lys Gln Gly Asn Trp
        35                  40                  45

cat gtc aat gat cga tcg cat tac ttg ata aat gaa gag gat ccg aaa     192
His Val Asn Asp Arg Ser His Tyr Leu Ile Asn Glu Glu Asp Pro Lys
    50                  55                  60

tta att tca tta aac aaa aca aat gaa gga att gct gtt att cct gga     240
Leu Ile Ser Leu Asn Lys Thr Asn Glu Gly Ile Ala Val Ile Pro Gly

65                  70                  75                  80

gca gaa aaa aac cag atc gtt act tcg aat tca act aaa tct ctt gat     288
Ala Glu Lys Asn Gln Ile Val Thr Ser Asn Ser Thr Lys Ser Leu Asp
                85                  90                  95

cag tta gat gta att ttt cca att gtt cat ggg acg ctc gga gaa gat     336
Gln Leu Asp Val Ile Phe Pro Ile Val His Gly Thr Leu Gly Glu Asp
            100                 105                 110

gga agc tta caa ggg atg ctt cga atg gca aac att cca ttt gtc gga     384
Gly Ser Leu Gln Gly Met Leu Arg Met Ala Asn Ile Pro Phe Val Gly
        115                 120                 125

tct agt gta ttg ggc tct gct att agc atg gat aaa gac att gcc aaa     432
Ser Ser Val Leu Gly Ser Ala Ile Ser Met Asp Lys Asp Ile Ala Lys
    130                 135                 140

cgt cta ttg aaa gac gct gga tta aaa gta gca aaa gga tat gct tat     480
Arg Leu Leu Lys Asp Ala Gly Leu Lys Val Ala Lys Gly Tyr Ala Tyr
145                 150                 155                 160

cga agt gtg gac aag gaa agc att cat ttt gat agt ctt aaa gaa gaa     528
Arg Ser Val Asp Lys Glu Ser Ile His Phe Asp Ser Leu Lys Glu Glu
                165                 170                 175

tta gga tta cct atg ttt att aaa cca gca aac cag ggt tcg tcg gtt     576
Leu Gly Leu Pro Met Phe Ile Lys Pro Ala Asn Gln Gly Ser Ser Val
            180                 185                 190

ggt gtt cac aaa gta gaa aac gaa gaa caa ttt tat tcg gct att aag     624
Gly Val His Lys Val Glu Asn Glu Glu Gln Phe Tyr Ser Ala Ile Lys
        195                 200                 205

gat gca ttt caa ttt gat cat aaa tta ctc gtt gaa gaa gca att gta     672
Asp Ala Phe Gln Phe Asp His Lys Leu Leu Val Glu Glu Ala Ile Val
    210                 215                 220

gga agg gaa att gaa tgt gct gtt ctg gga aat gaa aaa cca gct gca     720
Gly Arg Glu Ile Glu Cys Ala Val Leu Gly Asn Glu Lys Pro Ala Ala
225                 230                 235                 240

tct gtt cct gga gaa att tta ccg acg gga gat ttt tac tcc tat gaa     768
Ser Val Pro Gly Glu Ile Leu Pro Thr Gly Asp Phe Tyr Ser Tyr Glu
                245                 250                 255

gcg aaa tat att gat gaa acg ggg gca gta ctt caa att cct gct aaa     816
Ala Lys Tyr Ile Asp Glu Thr Gly Ala Val Leu Gln Ile Pro Ala Lys
            260                 265                 270

cta gaa gaa cct gta gta gac agt ata caa gat att gct tta caa gct     864
Leu Glu Glu Pro Val Val Asp Ser Ile Gln Asp Ile Ala Leu Gln Ala
        275                 280                 285

ttt aag gca ttg aat tgt gaa ggt ctg gca aga gtt gat gtg ttt tta     912
Phe Lys Ala Leu Asn Cys Glu Gly Leu Ala Arg Val Asp Val Phe Leu
    290                 295                 300

acg gat gac aac gaa att gta atc aat gaa ata aat aca ctt cca ggt     960
Thr Asp Asp Asn Glu Ile Val Ile Asn Glu Ile Asn Thr Leu Pro Gly
305                 310                 315                 320

ttt aca aaa ata agc atg tat cca aaa tta tgg gaa gaa agc gga gta     1008
Phe Thr Lys Ile Ser Met Tyr Pro Lys Leu Trp Glu Glu Ser Gly Val
                325                 330                 335

tct tat cgt gaa tta atc gag aat tta ata gaa ctt gct ata gag aga     1056
Ser Tyr Arg Glu Leu Ile Glu Asn Leu Ile Glu Leu Ala Ile Glu Arg

340                 345                 350

aat caa cgt gac caa caa tta aag agt tct aga agt gat gga          1098
Asn Gln Arg Asp Gln Gln Leu Lys Ser Ser Arg Ser Asp Gly
        355                 360                 365

<210> 10
<211> 909
<212> DNA
<213> Thermotoga maritima ATCC 43589

<400> 10
gtg aga gtg gct ctc ctg atg gga gga gtg tcg agg gaa agg gaa atc      48
Met Arg Val Ala Leu Leu Met Gly Gly Val Ser Arg Glu Arg Glu Ile
1               5                   10                  15

tct ctg aga agc gga gaa agg gta aaa aag gct ctt gaa aaa ctc ggg      96
Ser Leu Arg Ser Gly Glu Arg Val Lys Lys Ala Leu Glu Lys Leu Gly
            20                  25                  30

tat gaa cac acc gtt ttt gac gtt aga gaa gat ttt ctc aag aaa gtg     144
Tyr Glu His Thr Val Phe Asp Val Arg Glu Asp Phe Leu Lys Lys Val
        35                  40                  45

gat cag ttg aaa agt ttt gat gtg gtc ttc aac gtt ctc cat gga act     192
Asp Gln Leu Lys Ser Phe Asp Val Val Phe Asn Val Leu His Gly Thr
    50                  55                  60

ttc ggt gag gat gga acg ctt cag gca att ctg gat ttt ttg ggg atc     240
Phe Gly Glu Asp Gly Thr Leu Gln Ala Ile Leu Asp Phe Leu Gly Ile
65                  70                  75                  80

aga tac acc ggg tcc gat gcg ttt tca agc atg ata tgt ttt gat aaa     288
Arg Tyr Thr Gly Ser Asp Ala Phe Ser Ser Met Ile Cys Phe Asp Lys
                85                  90                  95

ctc gtg act tac agg ttt tta aag ggc acc gtc gag ata cct gat ttt     336
Leu Val Thr Tyr Arg Phe Leu Lys Gly Thr Val Glu Ile Pro Asp Phe
            100                 105                 110

gtg gag atc aag gaa ttc atg aaa act tct ccc ctt ggg tat ccc tgt     384
Val Glu Ile Lys Glu Phe Met Lys Thr Ser Pro Leu Gly Tyr Pro Cys
        115                 120                 125

gtg gta aaa ccg agg aga gag ggt tca agc ata gga gtg ttc gtt tgt     432
Val Val Lys Pro Arg Arg Glu Gly Ser Ser Ile Gly Val Phe Val Cys
    130                 135                 140

gaa tcg gat gaa gag ttt caa cac gcg ctc aag gaa gat tta cca cga     480
Glu Ser Asp Glu Glu Phe Gln His Ala Leu Lys Glu Asp Leu Pro Arg
145                 150                 155                 160

tat ggg agt gtg att gtt caa aag tac atc ccc ggc agg gaa atg act     528
Tyr Gly Ser Val Ile Val Gln Lys Tyr Ile Pro Gly Arg Glu Met Thr
                165                 170                 175

gtg tct att tta gaa acg gaa aag ggc ttc gaa att ctt ccc gtt ctt     576
Val Ser Ile Leu Glu Thr Glu Lys Gly Phe Glu Ile Leu Pro Val Leu
            180                 185                 190

gag ttg cga cca aag cgt cgt ttc tac gat tac gtt gcg aag tac acg     624
Glu Leu Arg Pro Lys Arg Arg Phe Tyr Asp Tyr Val Ala Lys Tyr Thr
        195                 200                 205

aaa ggt gaa acg gag ttc att ctt cct gct cct ttg aat cct tca gaa     672
Lys Gly Glu Thr Glu Phe Ile Leu Pro Ala Pro Leu Asn Pro Ser Glu
    210                 215                 220

gaa cgc ctt gtc aag gaa atg gct ctc aaa gcg ttt gta gaa gct ggt     720
Glu Arg Leu Val Lys Glu Met Ala Leu Lys Ala Phe Val Glu Ala Gly
225                 230                 235                 240

tgc aga ggt ttt ggg agg gtg gat ggt att ttc agc gat ggc agg ttc     768
Cys Arg Gly Phe Gly Arg Val Asp Gly Ile Phe Ser Asp Gly Arg Phe
                245                 250                 255

tac ttt ctg gaa atc aac acg gta ccg ggt cta acg gaa acc agt gat     816
Tyr Phe Leu Glu Ile Asn Thr Val Pro Gly Leu Thr Glu Thr Ser Asp
            260                 265                 270

ctc cca gcg agc gcg aaa gcg gga gga atc gag ttc gag gaa ctc gtt     864
Leu Pro Ala Ser Ala Lys Ala Gly Gly Ile Glu Phe Glu Glu Leu Val
        275                 280                 285

gag atc atc tta aag agc gct ttt ttg aaa gaa ggg gtg aga gta         909
Glu Ile Ile Leu Lys Ser Ala Phe Leu Lys Glu Gly Val Arg Val
    290                 295                 300


<210> 11
<211> 409
<212> PRT
<213> Pseudomonas putida KT2440(ATCC47954)

<400> 11
Met Pro Phe Arg Arg Thr Leu Leu Ala Ala Ser Leu Ala Leu Leu Ile
1               5                   10                  15

Thr Gly Gln Ala Pro Leu Tyr Ala Ala Pro Pro Leu Ser Met Asp Asn
            20                  25                  30

Gly Thr Asn Thr Leu Thr Val Gln Asn Ser Asn Ala Trp Val Glu Val
        35                  40                  45

Ser Ala Ser Ala Leu Gln His Asn Ile Arg Thr Leu Gln Ala Glu Leu
    50                  55                  60

Ala Gly Lys Ser Lys Leu Cys Ala Val Leu Lys Ala Asp Ala Tyr Gly
65                  70                  75                  80

His Gly Ile Gly Leu Val Met Pro Ser Ile Ile Ala Gln Gly Val Pro
                85                  90                  95

Cys Val Ala Val Ala Ser Asn Glu Glu Ala Arg Val Val Arg Ala Ser
            100                 105                 110

Gly Phe Thr Gly Gln Leu Val Arg Val Arg Leu Ala Ser Leu Ser Glu
        115                 120                 125

Leu Glu Asp Gly Leu Gln Tyr Asp Met Glu Glu Leu Val Gly Ser Ala
    130                 135                 140

Glu Phe Ala Arg Gln Ala Asp Ala Ile Ala Ala Arg His Gly Lys Thr
145                 150                 155                 160

Leu Arg Ile His Met Ala Leu Asn Ser Ser Gly Met Ser Arg Asn Gly
                165                 170                 175

Val Glu Met Ala Thr Trp Ser Gly Arg Gly Glu Ala Leu Gln Ile Thr
            180                 185                 190

Asp Gln Lys His Leu Lys Leu Val Ala Leu Met Thr His Phe Ala Val
        195                 200                 205

Glu Asp Lys Asp Asp Val Arg Lys Gly Leu Ala Ala Phe Asn Glu Gln

210                 215                 220

Thr Asp Trp Leu Ile Lys His Ala Arg Leu Asp Arg Ser Lys Leu Thr
225                 230                 235                 240

Leu His Ala Ala Asn Ser Phe Ala Thr Leu Glu Val Pro Glu Ala Arg
                245                 250                 255

Leu Asp Met Val Arg Thr Gly Gly Ala Leu Phe Gly Asp Thr Val Pro
            260                 265                 270

Ala Arg Thr Glu Tyr Lys Arg Ala Met Gln Phe Lys Ser His Val Ala
        275                 280                 285

Ala Val His Ser Tyr Pro Ala Gly Asn Thr Val Gly Tyr Asp Arg Thr
    290                 295                 300

Phe Thr Leu Ala Arg Asp Ser Arg Leu Ala Asn Ile Thr Val Gly Tyr
305                 310                 315                 320

Ser Asp Gly Tyr Arg Arg Val Phe Thr Asn Lys Gly His Val Leu Ile
                325                 330                 335

Asn Gly His Arg Val Pro Val Val Gly Lys Val Ser Met Asn Thr Leu
            340                 345                 350

Met Val Asp Val Thr Asp Phe Pro Asp Val Lys Gly Gly Asn Glu Val
        355                 360                 365

Val Leu Phe Gly Lys Gln Ala Gly Gly Glu Ile Thr Gln Ala Glu Met
    370                 375                 380

Glu Glu Ile Asn Gly Ala Leu Leu Ala Asp Leu Tyr Thr Val Trp Gly
385                 390                 395                 400

Asn Ser Asn Pro Lys Ile Leu Val Asp
                405


<210> 12
<211> 409
<212> PRT
<213> Pseudomonas putida IFO12996

<400> 12
Met Gln Phe Ser Arg Thr Leu Leu Ala Ala Ser Leu Ala Leu Leu Ile
1               5                   10                  15

Thr Gly Gln Ala Pro Leu Tyr Ala Ala Pro Pro Leu Ser Met Asp Asn
            20                  25                  30

Gly Thr Thr Ala Leu Thr Ala Gln Asn Ser Asn Ala Trp Val Glu Ile
        35                  40                  45

Ser Ala Gly Ala Leu Gln His Asn Ile Arg Thr Leu Gln Ala Glu Leu
    50                  55                  60

Gly Gly Lys Ser Lys Leu Cys Ala Val Leu Lys Ala Asp Ala Tyr Gly
65                  70                  75                  80

His Gly Ile Gly Leu Val Met Pro Ser Ile Ile Ala Gln Gly Val Pro
                85                  90                  95

Cys Val Ala Val Ala Ser Asn Glu Glu Ala Arg Val Val Arg Ala Ser
            100                 105                 110

Gly Phe Thr Gly Gln Leu Val Arg Val Arg Leu Ala Ser Leu Gly Glu
        115                 120                 125

Val Glu Asp Ala Leu Gln Tyr Asp Met Glu Glu Leu Val Gly Ser Ala
    130                 135                 140

Glu Phe Ala Arg Gln Leu Asp Ala Ile Ala Glu Arg His Gly Lys Thr
145                 150                 155                 160

Leu Arg Ile His Met Ala Leu Asn Ser Ser Gly Met Ser Arg Asn Gly
                165                 170                 175

Val Glu Met Thr Thr Trp Ser Gly Arg Gly Glu Ala Leu Gln Ile Thr
            180                 185                 190

Asp Gln Lys His Leu Gln Leu Val Ala Leu Met Thr His Phe Ala Val
        195                 200                 205

Glu Asp Lys Asp Asp Val Arg Lys Gly Leu Ala Ala Phe Asn Glu Gln
    210                 215                 220

Thr Asp Trp Leu Ile Lys His Ala Lys Leu Asp Arg Ser Lys Leu Thr
225                 230                 235                 240

Leu His Ala Ala Asn Ser Phe Ala Thr Leu Glu Val Pro Glu Ala His
                245                 250                 255

Leu Asp Met Val Arg Thr Gly Gly Ala Leu Phe Gly Asp Thr Val Pro
            260                 265                 270

Thr Arg Thr Glu Tyr Gln Arg Val Met Gln Phe Lys Ser His Val Ala
        275                 280                 285

Ala Val His Ser Tyr Pro Ala Gly Asn Thr Val Gly Tyr Asp Arg Thr
    290                 295                 300

Phe Thr Leu Ala Arg Asp Ser Arg Leu Ala Asn Ile Thr Val Gly Tyr
305                 310                 315                 320

Ser Asp Gly Tyr Arg Arg Val Phe Thr Asn Lys Gly His Val Leu Ile
                325                 330                 335

Asn Gly His Arg Val Pro Val Val Gly Lys Val Ser Met Asn Thr Leu
            340                 345                 350

Met Val Asp Val Thr Asp Phe Pro Asp Val Lys Gly Gly Asn Glu Val
        355                 360                 365

Val Leu Phe Gly Lys Gln Ala Gly Arg Glu Ile Thr Gln Ala Glu Ile
    370                 375                 380

Glu Glu Ile Asn Gly Ala Leu Leu Ala Asp Leu Tyr Thr Val Trp Gly
385                 390                 395                 400

Ser Ser Asn Pro Lys Ile Leu Val Asp
                405

<210> 13
<211> 1227
<212> DNA
<213> Pseudomonas putida KT2440(ATCC47954)

<400> 13
atg ccc ttt cgc cgt acc ctt ctg gct gca tcc ctg gca ctt ctg atc      48
Met Pro Phe Arg Arg Thr Leu Leu Ala Ala Ser Leu Ala Leu Leu Ile
1               5                   10                  15

acc gga cag gcc ccc ctg tat gcg gca cca ccg ttg tcg atg gac aac      96
Thr Gly Gln Ala Pro Leu Tyr Ala Ala Pro Pro Leu Ser Met Asp Asn

            20                  25                  30

ggc acc aac acc ctg acc gtg caa aac agc aat gcc tgg gtc gaa gtc 144
Gly Thr Asn Thr Leu Thr Val Gln Asn Ser Asn Ala Trp Val Glu Val
        35                  40                  45

agc gcc agc gcc ctg cag cac aac atc cgc acg ctg cag gcc gag ctg 192
Ser Ala Ser Ala Leu Gln His Asn Ile Arg Thr Leu Gln Ala Glu Leu
    50                  55                  60

gcc ggc aag tcc aag ctg tgc gcc gtg ctc aag gcc gat gcc tat ggc 240
Ala Gly Lys Ser Lys Leu Cys Ala Val Leu Lys Ala Asp Ala Tyr Gly
65                  70                  75                  80

cac ggt atc ggc ctg gta atg cca tcg atc atc gcc caa ggc gtg ccc 288
His Gly Ile Gly Leu Val Met Pro Ser Ile Ile Ala Gln Gly Val Pro
                85                  90                  95

tgc gtg gcg gtg gcc agc aac gag gag gcc cgc gtg gtc cgc gcc agt 336
Cys Val Ala Val Ala Ser Asn Glu Glu Ala Arg Val Val Arg Ala Ser
            100                 105                 110

ggc ttc acc ggg caa ctg gtg cgg gta cgc ctg gcc agc ctc agc gag 384
Gly Phe Thr Gly Gln Leu Val Arg Val Arg Leu Ala Ser Leu Ser Glu
        115                 120                 125

ctg gaa gat ggc ttg cag tac gac atg gaa gag ctg gtg ggc agc gcg 432
Leu Glu Asp Gly Leu Gln Tyr Asp Met Glu Glu Leu Val Gly Ser Ala
    130                 135                 140

gaa ttt gcc cgc cag gcc gat gcc atc gcc gcg cgc cat ggc aag acc 480
Glu Phe Ala Arg Gln Ala Asp Ala Ile Ala Ala Arg His Gly Lys Thr
145                 150                 155                 160

ttg cgc att cac atg gcg ctc aac tcc agc ggc atg agc cgc aac ggg 528
Leu Arg Ile His Met Ala Leu Asn Ser Ser Gly Met Ser Arg Asn Gly
                165                 170                 175

gtg gag atg gcc acc tgg tcc ggc cgt ggc gaa gcg ctg cag atc acc 576
Val Glu Met Ala Thr Trp Ser Gly Arg Gly Glu Ala Leu Gln Ile Thr
            180                 185                 190

gac cag aag cac ctc aag ctg gtc gcg ctg atg acc cac ttc gcc gtg 624
Asp Gln Lys His Leu Lys Leu Val Ala Leu Met Thr His Phe Ala Val
        195                 200                 205

gaa gac aag gac gat gta cgc aag ggc ctg gcg gca ttc aac gag cag 672
Glu Asp Lys Asp Asp Val Arg Lys Gly Leu Ala Ala Phe Asn Glu Gln
    210                 215                 220

acc gac tgg ttg atc aag cac gcc agg ctg gac cgc agc aag ctc acc 720
Thr Asp Trp Leu Ile Lys His Ala Arg Leu Asp Arg Ser Lys Leu Thr
225                 230                 235                 240

ctg cac gcc gcc aac tcg ttc gct acg ctg gaa gtg ccg gaa gcg cgc 768
Leu His Ala Ala Asn Ser Phe Ala Thr Leu Glu Val Pro Glu Ala Arg
                245                 250                 255

ctg gac atg gta cga acg ggt ggc gcg ctg ttc ggc gac acc gtg ccg 816
Leu Asp Met Val Arg Thr Gly Gly Ala Leu Phe Gly Asp Thr Val Pro
            260                 265                 270

gcg cgc acc gag tac aaa cgt gcg atg cag ttc aaa tcg cac gtg gcg 864
Ala Arg Thr Glu Tyr Lys Arg Ala Met Gln Phe Lys Ser His Val Ala
        275                 280                 285

gcg gtg cac agc tat ccg gcc ggc aac acc gtg ggc tat gac cgc acc 912
Ala Val His Ser Tyr Pro Ala Gly Asn Thr Val Gly Tyr Asp Arg Thr

290                 295                 300

ttc acc ctg gcc cgt gat tcg cgg ctg gcc aac att acg gtc ggg tac     960
Phe Thr Leu Ala Arg Asp Ser Arg Leu Ala Asn Ile Thr Val Gly Tyr
305                 310                 315                 320

tcc gat ggc tac cgc cgg gta ttc acc aac aag ggc cat gtg ctg atc     1008
Ser Asp Gly Tyr Arg Arg Val Phe Thr Asn Lys Gly His Val Leu Ile
                325                 330                 335

aac ggc cac cgt gtg ccg gtc gtg ggc aag gtg tcg atg aac acg ctg     1056
Asn Gly His Arg Val Pro Val Val Gly Lys Val Ser Met Asn Thr Leu
            340                 345                 350

atg gtc gat gtc acc gac ttc cct gat gtg aag ggg ggt aac gaa gtg     1104
Met Val Asp Val Thr Asp Phe Pro Asp Val Lys Gly Gly Asn Glu Val
        355                 360                 365

gtg ctg ttc ggc aag cag gcc ggg ggc gaa atc acc cag gcc gag atg     1152
Val Leu Phe Gly Lys Gln Ala Gly Gly Glu Ile Thr Gln Ala Glu Met
    370                 375                 380

gaa gaa atc aac ggc gcg ttg ctc gcc gat ttg tac acc gta tgg ggc     1200
Glu Glu Ile Asn Gly Ala Leu Leu Ala Asp Leu Tyr Thr Val Trp Gly
385                 390                 395                 400

aat tcc aac ccg aag ata ctc gtc gac                                 1227
Asn Ser Asn Pro Lys Ile Leu Val Asp
                405

<210> 14
<211> 1227
<212> DNA
<213> Pseudomonas putida IFO12996

<400> 14
atg caa ttt agc cgt acc ctc ctg gct gca tcc ctc gct ctg ctg atc     48
Met Gln Phe Ser Arg Thr Leu Leu Ala Ala Ser Leu Ala Leu Leu Ile
  1               5                  10                  15

act ggc cag gcc ccg ctg tac gcc gca ccg ccc ctg tcg atg gac aac     96
Thr Gly Gln Ala Pro Leu Tyr Ala Ala Pro Pro Leu Ser Met Asp Asn
            20                  25                  30

ggc acc acc gcc ctg acc gcg cag aac agc aac gcc tgg gtc gaa atc     144
Gly Thr Thr Ala Leu Thr Ala Gln Asn Ser Asn Ala Trp Val Glu Ile
        35                  40                  45

agt gcc ggc gca ctg caa cac aac atc cgt acc ttg cag gcc gag ttg     192
Ser Ala Gly Ala Leu Gln His Asn Ile Arg Thr Leu Gln Ala Glu Leu
    50                  55                  60

ggc ggc aag tcc aag ctg tgc gcc gtg ctc aag gcc gac gcc tat ggc     240
Gly Gly Lys Ser Lys Leu Cys Ala Val Leu Lys Ala Asp Ala Tyr Gly
65                  70                  75                  80

cac ggt atc ggc ctg gtg atg ccg tcg atc atc gcc cag ggc gtg ccc     288
His Gly Ile Gly Leu Val Met Pro Ser Ile Ile Ala Gln Gly Val Pro
                85                  90                  95

tgc gtg gcg gtg gcc agc aac gag gag gca cgc gtg gtc cgc gcc agt     336
Cys Val Ala Val Ala Ser Asn Glu Glu Ala Arg Val Val Arg Ala Ser
            100                 105                 110

ggc ttc acc ggg caa ctg gtg cgg gta cgc ctg gcc agc ctc ggc gaa     384
Gly Phe Thr Gly Gln Leu Val Arg Val Arg Leu Ala Ser Leu Gly Glu
        115                 120                 125

gtg gaa gat gcc ttg cag tac gac atg gaa gag ctg gtt ggc agc gcc     432
Val Glu Asp Ala Leu Gln Tyr Asp Met Glu Glu Leu Val Gly Ser Ala
    130                 135                 140

gag ttc gcc cgc cag ctc gat gcc atc gcc gaa cgc cac ggc aag acc     480
Glu Phe Ala Arg Gln Leu Asp Ala Ile Ala Glu Arg His Gly Lys Thr
145                 150                 155                 160

ctg cgc att cac atg gcg ctc aat tcc agc ggc atg agc cgc aac ggc     528
Leu Arg Ile His Met Ala Leu Asn Ser Ser Gly Met Ser Arg Asn Gly
                165                 170                 175

gtg gaa atg acc acc tgg tcc ggc cgg ggt gaa gcg ctg cag atc act     576
Val Glu Met Thr Thr Trp Ser Gly Arg Gly Glu Ala Leu Gln Ile Thr
            180                 185                 190

gac cag aag cac ctc cag ctg gtc gcg ctg atg act cac ttc gcc gtg     624
Asp Gln Lys His Leu Gln Leu Val Ala Leu Met Thr His Phe Ala Val
        195                 200                 205

gaa gac aag gac gat gtg cgc aaa ggc ctg gca gcg ttc aac gaa cag     672
Glu Asp Lys Asp Asp Val Arg Lys Gly Leu Ala Ala Phe Asn Glu Gln
    210                 215                 220

acc gac tgg ctg atc aag cac gcg aag ctt gat cgc agc aag ctc acc     720
Thr Asp Trp Leu Ile Lys His Ala Lys Leu Asp Arg Ser Lys Leu Thr
225                 230                 235                 240

ctg cat gcc gcc aac tcc ttc gct acg ctg gaa gtg ccg gaa gcg cac     768
Leu His Ala Ala Asn Ser Phe Ala Thr Leu Glu Val Pro Glu Ala His
                245                 250                 255

ctg gac atg gtg cgt acc ggt ggc gcg ctg ttc ggc gac acc gtg ccg     816
Leu Asp Met Val Arg Thr Gly Gly Ala Leu Phe Gly Asp Thr Val Pro
            260                 265                 270

acg cgc acc gaa tac caa cgt gtc atg cag ttc aag tcg cac gtg gcg     864
Thr Arg Thr Glu Tyr Gln Arg Val Met Gln Phe Lys Ser His Val Ala
        275                 280                 285

gcg gtg cac agc tac ccg gca ggc aac acc gtc ggc tac gac cgc acc     912
Ala Val His Ser Tyr Pro Ala Gly Asn Thr Val Gly Tyr Asp Arg Thr
    290                 295                 300

ttc acc ctg gcg cgt gat tcg cgc ctg gcc aac atc acc gtg ggt tac     960
Phe Thr Leu Ala Arg Asp Ser Arg Leu Ala Asn Ile Thr Val Gly Tyr
305                 310                 315                 320

tcc gat ggc tac cgc cgg gtg ttc acc aac aag ggc cat gtg ctg atc     1008
Ser Asp Gly Tyr Arg Arg Val Phe Thr Asn Lys Gly His Val Leu Ile
                325                 330                 335

aac ggc cac cga gtg cca gtg gtg ggc aag gtg tcg atg aac acc ttg     1056
Asn Gly His Arg Val Pro Val Val Gly Lys Val Ser Met Asn Thr Leu
            340                 345                 350

atg gtc gat gtc acc gat ttc ccc gat gtg aag ggg ggc aac gaa gtg     1104
Met Val Asp Val Thr Asp Phe Pro Asp Val Lys Gly Gly Asn Glu Val
        355                 360                 365

gtg ctg ttc ggc aaa cag gcc ggg agg gag atc acc cag gcc gag ata     1152
Val Leu Phe Gly Lys Gln Ala Gly Arg Glu Ile Thr Gln Ala Glu Ile
    370                 375                 380

gaa gaa atc aac ggc gcg ctg ctc gcc gac ctc tac acc gta tgg ggc     1200
Glu Glu Ile Asn Gly Ala Leu Leu Ala Asp Leu Tyr Thr Val Trp Gly
385                 390                 395                 400

agt tcc aac ccg aag att ctc gtc gac                                    1227
Ser Ser Asn Pro Lys Ile Leu Val Asp
                405

<210> 15
<211> 26
<212> DNA
<213> Artificial Sequence

<220>
<223> Description of Artificial Sequence: Synthetic DNA

<400> 15
gcccatatga ctgataaaat cgcggt                                            26

<210> 16
<211> 25
<212> DNA
<213> Artificial Sequence

<220>
<223> Description of Artificial Sequence: Synthetic DNA

<400> 16
gccaagcttt tagtccgcca gttcc                                             25

<210> 17
<211> 33
<212> DNA
<213> Artificial Sequence

<220>
<223> Description of Artificial Sequence: Synthetic DNA

<400> 17
ggtattgagg gtcgcatgac tgataaaatc gcg                                    33

<210> 18
<211> 30
<212> DNA
<213> Artificial Sequence

<220>
<223> Description of Artificial Sequence: Synthetic DNA

<400> 18
agaggagagt tagagcctta gtccgccagt                                        30

<210> 19
<211> 33
<212> DNA
<213> Artificial Sequence

<220>
<223> Description of Artificial Sequence: Synthetic DNA

<400> 19
ggtattgagg gtcgcatgga aaaactgcgg ggt                                    33

<210> 20
<211> 30

<212> DNA
<213> Artificial Sequence

<220>
<223> Description of Artificial Sequence: Synthetic DNA

<400> 20
agaggagagt tagagcctta cattgtggtt                                        30


<210> 21
<211> 32
<212> DNA
<213> Artificial Sequence

<220>
<223> Description of Artificial Sequence: Synthetic DNA

<400> 21
ggtattgagg gtcgcatgaa gaaaaaagtt gg                                      32


<210> 22
<211> 30
<212> DNA
<213> Artificial Sequence

<220>
<223> Description of Artificial Sequence: Synthetic DNA

<400> 22
agaggagagt tagagcccta tccatcactt                                        30


<210> 23
<211> 28
<212> DNA
<213> Artificial Sequence

<220>
<223> Description of Artificial Sequence: Synthetic DNA

<400> 23
ggtattgagg gtcgcatgcg tgtggggc                                          28


<210> 24
<211> 33
<212> DNA
<213> Artificial Sequence

<220>
<223> Description of Artificial Sequence: Synthetic DNA

<400> 24
agaggagagt tagagcctta ttggtgaccg gga                                    33


<210> 25
<211> 25
<212> DNA
<213> Artificial Sequence

<220>
<223> Description of Artificial Sequence: Synthetic DNA

<400> 25
ccgggatcca tgcaatttag ccgta                                             25

<210> 26
<211> 30
<212> DNA
<213> Artificial Sequence

<220>
<223> Description of Artificial Sequence: Synthetic DNA

<400> 26
ccggaattct cagtcgacga gaatcttcgg                                        30


<210> 27
<211> 28
<212> DNA
<213> Artificial Sequence

<220>
<223> Description of Artificial Sequence: Synthetic DNA

<400> 27
ggaacatgtt gtttagccgt accctcct                                          28


<210> 28
<211> 20
<212> DNA
<213> Artificial Sequence

<220>
<223> Description of Artificial Sequence: Synthetic DNA

<400> 28
ggcgaattcg tcgacgagat                                                   20


<210> 29
<211> 30
<212> DNA
<213> Artificial Sequence

<220>
<223> Description of Artificial Sequence: Synthetic DNA

<400> 29
ggtattgagg gtcgcatgag agtggctctc                                        30


<210> 30
<211> 34
<212> DNA
<213> Artificial Sequence

<220>
<223> Description of Artificial Sequence: Synthetic DNA

<400> 30
agaggagagt tagagcctca tactctcacc cctt                                   34

## Description

### Technical Field

The present invention relates to an enzymatic process for producing a dipeptide.

### Background Art

Known methods for producing dipeptides comprising D-amino acid include chemical synthesis and enzymatic methods. In the synthesis of dipeptides comprising D-amino acid by the chemical synthesis methods, operations such as introduction and removal of protective groups for functional groups are necessary, and racemates are also formed. The chemical synthesis methods are thus considered to be disadvantageous in respect of cost and efficiency. They are unfavorable also from the viewpoint of environmental hygiene because of the use of large amounts of organic solvents and the like.

As to the synthesis of dipeptides comprising D-amino acid by the enzymatic methods, the following methods are known: a method utilizing reverse reaction of peptidase (see non-patent publication No. 1); methods utilizing aminoacyl transferase (see non-patent publication Nos. 2 and 3); and a method utilizing α-amino acid esterase (see non-patent publication No. 4).

However, the method utilizing reverse reaction of peptidase requires introduction and removal of protective groups for functional groups of amino acids used as substrates, which causes difficulties in raising the efficiency of reaction to form dipeptides comprising D-amino acid and in preventing dipeptide hydrolysis reaction. The methods utilizing aminoacyl transferase are not efficient, for they require the process of esterification of amino acids used as substrates. The method utilizing α-amino acid esterase has the defect that it also requires the process of esterification of amino acids used as substrates, which causes difficulties in raising the efficiency of reaction to form dipeptides comprising D-amino acid and in preventing peptide hydrolysis reaction.

D-alanine-D-alanine ligase is known to have the activity to form D-alanyl-D-alanine and D-alanyl-D-serine using D-alanine, and D-alanine and D-serine, as respective substrates (see non-patent publication No. 5). However, it is not known that the enzyme has the activity to form dipeptides comprising D-amino acid other than D-alanyl-D-alanine and D-alanyl-D-serine, and a process for producing dipeptides comprising D-amino acid other than D-alanyl-D-alanine and D-alanyl-D-serine using, as an enzyme source, a culture of a microorganism having the ability to produce the enzyme is not known either.

Known examples of proteins having the activity to form D-amino acid include hydantoinase having the activity to form D-amino acid from 5-substituted-DL-hydantoin (see non-patent publication No. 6), D-aminoacylase having the activity to form D-amino acid from N-acyl-DL-amino acid (see non-patent publication No. 7), D-amino-acid amidase having the activity to form D-amino acid from DL-amino acid amide (see non-patent publication No. 8), D-amino-acid aminotransferase having the activity to form D-amino acid from α-keto acid (see non-patent publication No. 9) and amino-acid racemase having the activity to form D-amino acid from L-α-amino acid. Low-substrate-specific amino-acid racemase, which is classified as EC 5.1.1.10 and whose substrate specificity is very low, is an enzyme useful for the industrial production of racemates of various amino acids. The microorganisms so far reported to produce this enzyme are microorganisms belonging to the genera Pseudomonas (see non-patent publication Nos. 10 and 11) and Aeromonas (see non-patent publication No. 12). However, their activity is often weak, and it is difficult to fully compensate this defect by improvement of culturing method or acquisition of a mutant strain.

With regard to the proteins having low-substrate-specific amino-acid racemase activity, there is a report on the amino acid sequence of the active site of the protein derived from Pseudomonas putida IFO12996 (see non-patent publication No. 13). There is also a report that DNA encoding the enzyme was obtained and introduced into a microorganism such as Escherichia coli to express a protein having the enzyme activity (see patent publication No. 1).

However, it is not known that dipeptides comprising D-amino acid are formed from amino acids including L-α-amino acid by using a protein having amino-acid racemase activity and a protein having D-alanine-D-alanine ligase activity in combination.
Non-patent publication No. 1:
   J. Mol. Cat. B Enzymatic, 6, 379-386 (1999)
Non-patent publication No. 2:
   J. Biochem., 131, 247-254 (2002)
Non-patent publication No. 3:
   J. Biosci. Bioeng., 89, 195-306 (2000)
Non-patent publication No. 4:
   J. Biochem., 130, 119-126 (2001)
Non-patent publication No. 5:
   Chem. Biol., 5, 197-207 (1998)
Non-patent publication No. 6:
   Biotechnol. Bioeng., 23, 2173-2183 (1981)
Non-patent publication No. 7:
   Agric. Biol. Chem., 44, 1089-1095 (1980)
Non-patent publication No. 8:
   Biochem. Biophys. Res. Commun., 162, 470-474 (1989) Non-patent publication No. 9:
   FEBS Lett., 55, 265-267 (1975)
Non-patent publication No. 10:
   Methods Enzymol., 17B, 629-636 (1971)
Non-patent publication No. 11:
   J. Bacteriol., 175, 4213-4217 (1993)
Non-patent publication No. 12:
   Agric. Biol. Chem., 51, 173-180 (1987)
Non-patent publication No. 13:
   Biochemistry, 23, 5195-5201 (1984)
Patent publication No. 1:
   WO03/074690 pamphlet

### Disclosure of the Invention

### Problems to be Solved by the Invention

An object of the present invention is to provide a process for producing a dipeptide other than D-alanyl-D-alanine and D-alanyl-D-serine using, as an enzyme source, a culture of a microorganism having the ability to produce a protein having D-alanine-D-alanine ligase activity or a treated matter of the culture, and a process for producing a dipeptide comprising D-amino acid using, as enzyme sources, a culture of a microorganism having the ability to produce the D-amino acid or a treated matter of the culture, and a culture of a microorganism having the ability to produce a protein having D-alanine-D-alanine ligase activity or a treated matter of the culture.

### Means for Solving the Problems

The present invention relates to the following (1) to (18).
(1) A process for producing a dipeptide, which comprises: allowing an enzyme source, ATP and one or more kinds of amino acids selected from the group consisting of D-amino acids and glycine (provided that said one or more kinds of amino acids are not D-alanine alone or a combination of D-alanine and D-serine) to be present in an aqueous medium, said enzyme source being a culture of a microorganism having the ability to produce a protein having D-alanine-D-alanine ligase activity or a treated matter of the culture; allowing the dipeptide to form and accumulate in the aqueous medium; and recovering the dipeptide from the aqueous medium.
(2) The process according to the above (1), wherein the D-amino acid is produced by using a culture of a microorganism having the ability to produce the D-amino acid or a treated matter of the culture as an enzyme source.
(3) A process for producing a dipeptide comprising D-amino acid which comprises: allowing enzyme sources, ATP and a substance which is converted into the D-amino acid by a culture of a microorganism having the ability to produce the D-amino acid or a treated matter of the culture to be present in an aqueous medium, said enzyme sources being a culture of a microorganism having the ability to produce the D-amino acid or a treated matter of the culture, and a culture of a microorganism having the ability to produce a protein having D-alanine-D-alanine ligase activity or a treated matter of the culture; allowing the dipeptide comprising D-amino acid to form and accumulate in the aqueous medium; and recovering the dipeptide from the aqueous medium.
(4) The process according to any one of the above (1) to (3), wherein the protein having D-alanine-D-alanine ligase activity is a protein selected from the group consisting of the following [1] to [3]:
   [1] a protein having the amino acid sequence shown in any of SEQ ID NOS: 1 to 5;
   [2] a protein consisting of an amino acid sequence wherein one or more amino acid residues are deleted, substituted or added in the amino acid sequence shown in any of SEQ ID NOS: 1 to 5 and having D-alanine-D-alanine ligase activity; and
   [3] a protein consisting of an amino acid sequence which has 80% or more homology to the amino acid sequence shown in any of SEQ ID NOS: 1 to 5 and having D-alanine-D-alanine ligase activity.
(5) The process according to any one of the above (1) to (3), wherein the microorganism having the ability to produce a protein having D-alanine-D-alanine ligase activity is a microorganism obtainable by introducing DNA encoding the protein having D-alanine-D-alanine ligase activity into a host cell.
(6) The process according to the above (5), wherein the DNA encoding a protein having D-alanine-D-alanine ligase activity is DNA selected from the group consisting of the following [1] to [4]:
   [1] DNA encoding a protein having the amino acid sequence shown in any of SEQ ID NOS: 1 to 5;
   [2] DNA having the nucleotide sequence shown in any of SEQ ID NOS: 6 to 10;
   [3] DNA which hybridizes with DNA having a nucleotide sequence complementary to the nucleotide sequence shown in any of SEQ ID NOS: 6 to 10 under stringent conditions and which encodes a protein having D-alanine-D-alanine ligase activity; and
   [4] DNA consisting of a nucleotide sequence which has 90% or more homology to the nucleotide sequence shown in any of SEQ ID NOS: 6 to 10 and encoding a protein having D-alanine-D-alanine ligase activity.
(7) The process according to the above (2) or (3), wherein the microorganism having the ability to produce D-amino acid is a microorganism having the ability to produce a protein having the activity of hydantoinase, D-aminoacylase, D-amino-acid amidase, D-amino-acid transaminase or amino-acid racemase.
(8) The process according to the above (7), wherein the protein having amino-acid racemase activity is a protein having low-substrate-specific amino-acid racemase activity.
(9) The process according to the above (8), wherein the protein having low-substrate-specific amino-acid racemase activity is a protein selected from the group consisting of the following [1] to [3]:
   [1] a protein having the amino acid sequence shown in SEQ ID NO: 11 or 12;
   [2] a protein consisting of an amino acid sequence wherein one or more amino acid residues are deleted, substituted or added in the amino acid sequence shown in SEQ ID NO: 11 or 12 and having low-substrate-specific amino-acid racemase activity; and
   [3] a protein consisting of an amino acid sequence which has 80% or more homology to the amino acid sequence shown in SEQ ID NO: 11 or 12 and having low-substrate-specific amino-acid racemase activity.
(10) The process according to the above (2) and (3), wherein the microorganism having the ability to produce D-amino acid is a microorganism obtainable by introducing DNA encoding a protein having the activity of hydantoinase, D-aminoacylase, D-amino-acid amidase, D-amino-acid aminotransferase or amino-acid racemase into a host cell.
(11) The process according to the above (10), wherein the DNA encoding a protein having amino-acid racemase activity is DNA encoding low-substrate-specific amino-acid racemase.
(12) The process according to the above (11), wherein the DNA encoding a protein having low-substrate-specific amino-acid racemase activity is DNA selected from the group consisting of the following [1] to [4]:
   [1] DNA encoding a protein having the amino acid sequence shown in SEQ ID NO: 11 or 12;
   [2] DNA having the nucleotide sequence shown in SEQ ID NO: 13 or 14;
   [3] DNA which hybridizes with DNA having a nucleotide sequence complementary to the nucleotide sequence shown in SEQ ID NO: 13 or 14 under stringent conditions and which encodes a protein having low-substrate-specific amino-acid racemase activity; and
   [4] DNA consisting of a nucleotide sequence which has 90% or more homology to the nucleotide sequence shown in SEQ ID NO: 13 or 14 and encoding a protein having low-substrate-specific amino-acid racemase activity.
(13) The process according to any one of the above (1), (2) and (4) to (6), wherein the one or more kinds of amino acids selected from the group consisting of D-amino acids and glycine are D-amino acids selected from the group consisting of D-alanine, D-glutamine, D-glutamic acid, glycine, D-valine, D-leucine, D-isoleucine, D-proline, D-phenylalanine, D-tryptophan, D-methionine, D-serine, D-threonine, D-cysteine, D-asparagine, D-tyrosine, D-lysine, D-arginine, D-histidine, D-aspartic acid and D-ornithine (provided that said one or more kinds of amino acids are not D-alanine alone or a combination of D-alanine and D-serine).
(14) The process according to any one of the above (3) to (12), wherein the substance which is converted into D-amino acid by a culture of a microorganism having the ability to produce the D-amino acid or a treated matter of the culture is one or more kinds of 5-substituted-DL-hydantoin, one or more kinds of N-acyl-DL-amino acids, one or more kinds of DL-amino acid amides, one or more kinds of α-keto acids and one or more kinds of D-amino acids, or one or more kinds of amino acids comprising one or more kinds of L-α-amino acids.
(15) The process according to the above (14), wherein the one or more kinds of amino acids comprising one or more kinds of L-α-amino acids comprise one or more kinds of amino acids selected from the group consisting of L- or D-form of alanine, glutamine, glutamic acid, valine, leucine, isoleucine, proline, phenylalanine, tryptophan, methionine, serine, threonine, cysteine, asparagine, tyrosine, lysine, arginine, histidine, aspartic acid and ornithine, and glycine.
(16) The process according to any one of the above (1), (2) and (4) to (12), wherein the dipeptide is a dipeptide represented by formula (I):

   R¹ - R² (I)

   (wherein R¹ and R², which may be the same or different, each represent D-form of alanine, glutamine, glutamic acid, valine, leucine, isoleucine, proline, phenylalanine, tryptophan, methionine, serine, threonine, cysteine, asparagine, tyrosine, lysine, arginine, histidine, aspartic acid or ornithine, or glycine; provided that both R¹ and R² cannot represent D-alanine at the same time, and when R¹ is D-alanine, R² does not represent D-serine).
(17) The process according to any one of the above (3) to (12), wherein the dipeptide comprising D-amino acid is a dipeptide represented by formula (II):

   R³ - R⁴ (II)

   (wherein R³ and R⁴, which may be the same or different, each represent L- or D-form of alanine, glutamine, glutamic acid, valine, leucine, isoleucine, proline, phenylalanine, tryptophan, methionine, serine, threonine, cysteine, asparagine, tyrosine, lysine, arginine, histidine, aspartic acid or ornithine, or glycine; provided that both R³ and R⁴ cannot represent L-form of amino acids or glycine at the same time).
(18) The process according to any one of the above (3) to (12), wherein the dipeptide comprising D-amino acid is a dipeptide represented by formula (III):

   R⁵ - R⁶ (III)

   (wherein R⁵ and R⁶, which may be the same or different, each represent D-alanine, D-glutamine, D-glutamic acid, D-valine, D-leucine, D-isoleucine, D-proline, D-phenylalanine, D-tryptophan, D-methionine, D-serine, D-threonine, D-cysteine, D-asparagine, D-tyrosine, D-lysine, D-arginine, D-histidine, D-aspartic acid, D-ornithine or glycine; provided that both R⁵ and R⁶ cannot represent glycine at the same time).

### Effect of the Invention

The present invention provides a process for producing a dipeptide other than D-alanyl-D-alanine and D-alanyl-D-serine using, as an enzyme source, a culture of a microorganism having the ability to produce a protein having D-alanine-D-alanine ligase activity or a treated matter of the culture, and a process for producing a dipeptide comprising D-amino acid using, as enzyme sources, a culture of a microorganism having the ability to produce the D-amino acid or a treated matter of the culture, and a culture of a microorganism having the ability to produce a protein having D-alanine-D-alanine ligase activity or a treated matter of the culture.

### Best Modes for Carrying Out the Invention

The present invention is described in detail below.

### 1. Microorganisms Having the Ability to Produce a Protein Having D-Alanine-D-Alanine Ligase Activity Used in the Process of the Present Invention

The microorganisms having the ability to produce a protein having D-alanine-D-alanine ligase activity used in the process of the present invention may be any microorganisms that have the ability to produce a protein having D-alanine-D-alanine ligase activity.

Many microorganisms have D-alanine-D-alanine ligase (also referred to as ddl) activity, and examples of the microorganisms are those belonging to the genus Escherichia [described in DNA Data Bank of Japan (DDBJ) as having ddlB gene and ddlA gene], those belonging to the genus Oceanobacillus (described in DDBJ as having ddlA gene), those belonging to the genus Synechocystis (described in DDBJ as having ddlA gene), those belonging to the genus Bacillus [Nature, 390, 249-256 (1997)], those belonging to the genus Corynebacterium (D-alanine-D-alanine ligase and a gene encoding the enzyme are shown under NCBI ACCESSION NO. AP005282), those belonging to the genus Pseudomonas [Microbiology, 142, 79-86 (1996)], those belonging to the genus Helicobacter [Nature, 388, 539-547 (1997)], those belonging to the genus Mycobacterium [Nature, 393, 537-544 (1998)], those belonging to the genus Arabidopsis (the protein represented by NCBI ACCESSION NO. NM_202530 is described as belonging to the family of D-alanine-D-alanine ligase) and those belonging to the genus Thermotoga (described in DDBJ as having a gene encoding D-alanine-D-alanine ligase). Other microorganisms that produce a protein having D-alanine-D-alanine ligase activity can be obtained by searching various kinds of public databases with the name of the enzyme as a keyword.

Microorganisms preferred for use in the present invention are those belonging to the genera Escherichia, Oceanobacillus, Synechocystis and Thermotoga.

Specifically, the microorganisms having the ability to produce a protein having D-alanine-D-alanine ligase activity include the following [1] to [3]:
[1] a microorganism having the ability to produce a protein having the amino acid sequence shown in any of SEQ ID NOS: 1 to 5;
[2] a microorganism having the ability to produce a protein consisting of an amino acid sequence wherein one or more amino acid residues are deleted, substituted or added in the amino acid sequence shown in any of SEQ ID NOS: 1 to 5 and having low-substrate-specific amino-acid racemase activity; and
[3] a microorganism having the ability to produce a protein having 80% or more homology, preferably 90% or more homology, more preferably 95% or more homology, further preferably 98% or more homology, particularly preferably 99% or more homology to the amino acid sequence shown in any of SEQ ID NOS: 1 to 5 and having D-alanine-D-alanine ligase activity.

Specific examples of the microorganisms are Escherichia coli K-12, which is a microorganism having the ability to produce a protein having the amino acid sequence shown in SEQ ID NO: 1 or 2; Oceanobacillus iheyensis HTE831, which is a microorganism having the ability to produce a protein having the amino acid sequence shown in SEQ ID NO: 3; Synechocystis sp. PCC6803, which is a microorganism having the ability to produce a protein having the amino acid sequence shown in SEQ ID NO: 4; and Thermotoga maritima ATCC 43589, which is a microorganism having the ability to produce a protein having the amino acid sequence shown in SEQ ID NO: 5.

The above microorganisms having the ability to produce a protein consisting of an amino acid sequence wherein one or more amino acid residues are deleted, substituted or added and having D-alanine-D-alanine ligase activity include any microorganisms that have the ability to produce the protein, for example, those obtained by Southern analysis using, as a probe, a part or the whole of DNA having a nucleotide sequence complementary to DNA encoding a protein consisting of the amino acid sequence shown in any of SEQ ID NOS: 1 to 5, more specifically, DNA consisting of the nucleotide sequence shown in any of SEQ ID NOS: 6 to 10. An example of a part of DNA used as the above probe is DNA encoding the active center of a protein consisting of the amino acid sequence shown in any of SEQ ID NOS: 1 to 5.

The above Southern analysis refers to a process of carrying out Southern hybridization, using the above DNA as a probe, of a restriction enzyme digest of chromosomal DNA extracted from the cell of a test microorganism by a known method and checking the presence or absence of the positive signal. Specifically, the process is carried out in the following manner. Hybridization is carried out at 65°C in the presence of 0.7 to 1.0 mol/l sodium chloride using a filter with the restriction enzyme digest of the chromosomal DNA immobilized thereon, and the filter is washed at 65°C with a 0.1 to 2-fold conc. SSC solution (1-fold conc. SSC solution: 150 mmol/l sodium chloride and 15 mmol/l sodium citrate), followed by detection of DNA which hybridizes with the probe DNA even after washing. Hybridization can be carried out according to the methods described in Molecular Cloning, Third Edition; Current Protocols in Molecular Biology; DNA Cloning 1: Core Techniques, A Practical Approach, Second Edition, Oxford University (1995), etc. The microorganisms having hybridizable DNA include, for example, those belonging to the genera Escherichia, Oceanobacillus, Synechocystis and Thermotoga.

The microorganisms having the ability to produce a protein having 80% or more homology to the amino acid sequence shown in any of SEQ ID NOS: 1 to 5 and having D-alanine-D-alanine ligase activity include microorganisms having the ability to produce a protein which consists of an amino acid sequence having at least 80% homology, preferably 90% or more homology, more preferably 95% or more homology, further preferably 98% or more homology, particularly preferably 99% or more homology to the amino acid sequence shown in any of SEQ ID NOS: 1 to 5 as calculated by use of algorithm BLAST by Karlin and Altschul [Pro. Natl. Acad. Sci. USA, 90, 5873 (1993)] and FASTA [Methods Enzymol., 183, 63 (1990)] and which has D-alanine-D-alanine ligase activity. Examples of the microorganisms are those belonging to the genera Escherichia, Oceanobacillus, Synechocystis and Thermotoga.

On the basis of the above BLAST and FASTA, programs such as BLASTN and BLASTX have been developed [J. Mol. Biol., 215, 403 (1990)]. When a nucleotide sequence is analyzed by BLASTN on the basis of BLAST, the parameters, for instance, are as follows: score=100 and wordlength=12. When an amino acid sequence is analyzed by BLASTX on the basis of BLAST, the parameters, for instance, are as follows: score=50 and wordlength=3. The homology among nucleotide sequences and amino acid sequences can be calculated in this way. When BLAST and Gapped BLAST programs are used, default parameters of each program are used. The specific techniques for these analyses are known (http://www.ncbi.nlm.nih.gov.).

The microorganisms having the ability to produce a protein having D-alanine-D-alanine ligase activity used in the process of the present invention also include microorganisms which are obtained by treating the above microorganisms having the ability to produce a protein having D-alanine-D-alanine ligase activity by ordinary mutation-inducing means such as a treatment with a mutagen (e.g., N-methyl-N'-nitro-N-nitrosoguanidine) and mutation treatment by UV irradiation or γ-rays irradiation, and then selecting microorganisms showing higher D-alanine-D-alanine ligase activity compared with their parent strain. D-alanine-D-alanine ligase activity can be measured according to a conventional method.

Further, the microorganisms having the ability to produce a protein having D-alanine-D-alanine ligase activity used in the present invention include transformed microorganisms obtainable by introducing DNA encoding D-alanine-D-alanine ligase into a host cell.

The DNAs encoding a protein having D-alanine-D-alanine ligase activity include genes encoding D-alanine-D-alanine ligase (ddl genes) of the above microorganisms having the ability to produce a protein having D-alanine-D-alanine ligase activity which belong to the genera Escherichia, Oceanobacillus, Synechocystis, Bacillus, Corynebacterium, Pseudomonas, Helicobacter, Mycobacterium, Arabidopsis and Thermotoga. The specific nucleotide sequences of the genes can be obtained from various public databases. DNAs encoding D-alanine-D-alanine ligase preferably used in the present invention include DNAs encoding a protein having D-alanine-D-alanine ligase activity derived from microorganisms belonging to the genus Escherichia, Oceanobacillus, Synechocystis or Thermotoga. Specific examples of the DNAS encoding a protein having D-alanine-D-alanine ligase activity are the following [1] to [4]:
[1] DNA encoding a protein having the amino acid sequence shown in any of SEQ ID NOS: 1 to 5;
[2] DNA having the nucleotide sequence shown in any of SEQ ID NOS: 6 to 10;
[3] DNA which hybridizes with DNA having a nucleotide sequence complementary to the nucleotide sequence shown in any of SEQ ID NOS: 6 to 10 under stringent conditions and which encodes a protein having D-alanine-D-alanine ligase activity; and
[4] DNA consisting of a nucleotide sequence which has 90% or more homology, preferably 95% or more homology, more preferably 98% or more homology, further preferably 99% or more homology to the nucleotide sequence shown in any of SEQ ID NOS: 6 to 10 and encoding a protein having D-alanine-D-alanine ligase activity.

"To hybridize" refers to a step of hybridization of DNA with DNA having a specific nucleotide sequence or a part of the DNA. Therefore, the nucleotide sequence of the DNA having a specific nucleotide sequence or a part of the DNA may be DNA which is long enough to be useful as a probe for Northern or Southern blot analysis or to be used as an oligonucleotide primer for PCR analysis. Preferably, said DNA comprising at least 10, more preferably at least 15 nucleotides can be used as a probe while said DNA include the DNAs having at least 100 nucleotides, preferably 200 or more nucleotides, more preferably 500 or more nucleotides.

The method for hybridization of DNA is well known and the conditions for hybridization can be determined by a person skilled in the art according to the present specification. The hybridization can be carried out according to the methods described in Molecular Cloning, Second Edition, Third Edition (2001); Methods for General and Molecular Bacteriology, ASM Press (1994); Immunology methods manual, Academic press (Molecular), and many other standard textbooks.

Hybridization under the above stringent conditions is preferably carried out, for example, as follows. A filter with DNA immobilized thereon and a probe DNA are incubated in a solution comprising 50% formamide, 5 x SSC (750 mM sodium chloride and 75 mM sodium citrate), 50 mM sodium phosphate (pH 7.6), 5 x Denhardt's solution, 10% dextran sulfate and 20 µg/l denatured salmon sperm DNA at 42°C overnight, and after the incubation, the filter is washed in 0.2 x SSC solution (ca. 65°C). Less stringent conditions can also be employed. Modification of the stringent conditions can be made by adjusting the concentration of formamide (the conditions become less stringent as the concentration of formamide is lowered) and by changing the salt concentrations and the temperature conditions. Hybridization under less stringent conditions is carried out, for example, by incubating a filter with DNA immobilized thereon and a probe DNA in a solution comprising 6 x SSCE (20 x SSCE: 3 mol/l sodium chloride, 0.2 mol/l sodium dihydrogenphosphate and 0.02 mol/l EDTA, pH 7.4), 0.5% SDS, 30% formamide and 100 µg/l denatured salmon sperm DNA at 37°C overnight, and washing the filter with 1 x SSC solution containing 0.1% SDS (50°C). Hybridization under still less stringent conditions is carried out by using a solution having a high salt concentration (for example, 5 x SSC) under the above less stringent conditions, followed by washing.

Various conditions described above can also be established by adding a blocking reagent used to reduce the background of hybridization or changing the reagent. The addition of the above blocking reagent may be accompanied by changes of conditions for hybridization to make the conditions suitable for the purpose.

The above DNA capable of hybridization under stringent conditions includes DNA having at least 90% homology, preferably 95% or more homology, more preferably 98% or more homology, further preferably 99% or more homology to the nucleotide sequence shown in any of SEQ ID NOS: 6 to 10 as calculated by use of programs such as BLAST and FASTA described above based on the above parameters.

### 2. Microorganisms Having the Ability to Produce D-Amino Acid Used in the Process of the Present Invention

The microorganisms having the ability to produce D-amino acid used in the process of the present invention may be any microorganisms having such ability. Examples of microorganisms include those having the ability to produce hydantoinase having the activity to form D-amino acid from 5-substituted-DL-hydantoin [Biotechnol. Bioeng., 23, 2173-2183 (1981)], D-aminoacylase having the activity to form D-amino acid from N-acyl-DL-amino acid [Agric. Biol. Chem., 44, 1089-1095 (1980)], D-amino-acid amidase having the activity to form D-amino acid from DL-amino acid amide [Biochem. Biophys. Res. Commun., 162, 470-474 (1989)], D-amino-acid aminotransferase having the activity to form D-amino acid from α-keto acid [FEBS Lett., 55, 265-267 (1975)] or amino-acid racemase having the activity to form D-amino acid from L-α-amino acid. Preferred are microorganisms having the ability to produce amino-acid racemase, and more preferred are microorganisms having the ability to produce low-substrate-specific amino-acid racemase.

Examples of the microorganisms having the ability to produce amino-acid racemase include those belonging to the genera Escherichia [Science, 277, 1453-1474 (1997)], Bacillus [Nature, 390, 249-256 (1997)], Pseudomonas [Nature, 406, 959-964 (2000)] and Oceanobacillus [Nucleic Acids Res., 30, 3927-3935 (2002)], and examples of the microorganisms having the ability to produce a protein having low-substrate-specific amino-acid racemase activity include those belonging to the genus Pseudomonas (WO03/074690). Microorganisms having the ability to produce a protein having low-substrate-specific amino-acid racemase activity are preferably used in the present invention.

Specifically, the microorganisms having the ability to produce a protein having low-substrate-specific amino-acid racemase activity include the following [1] to [3]:
[1] a microorganism having the ability to produce a protein having the amino acid sequence shown in SEQ ID NO: 11 or 12;
[2] a microorganism having the ability to produce a protein consisting of an amino acid sequence wherein one or more amino acid residues are deleted, substituted or added in the amino acid sequence shown in SEQ ID NO: 11 or 12 and having low-substrate-specific amino-acid racemase activity; and
[3] a microorganism having the ability to produce a protein having 80% or more homology, preferably 90% or more homology, more preferably 95% or more homology, further preferably 98% or more homology, particularly preferably 99% or more homology to the amino acid sequence shown in SEQ ID NO: 11 or 12 and having low-substrate-specific amino-acid racemase activity.

Specific examples of the microorganisms are Pseudomonas putida ATCC 47954, which is a microorganism having the ability to produce a protein having the amino acid sequence shown in SEQ ID NO: 11, and Pseudomonas putida IFO 12996, which is a microorganism having the ability to produce a protein having the amino acid sequence shown in SEQ ID NO: 12.

The above microorganisms having the ability to produce a protein consisting of an amino acid sequence wherein one or more amino acid residues are deleted, substituted or added and having low-substrate-specific amino-acid racemase activity include any microorganisms that have the ability to produce the protein, for example, microorganisms obtained by Southern analysis using, as a probe, a part or the whole of DNA having a nucleotide sequence complementary to DNA encoding a protein consisting of the amino acid sequence shown in SEQ ID NO: 11 or 12, more specifically, DNA consisting of the nucleotide sequence shown in SEQ ID NO: 13 or 14. An example of a part of DNA used as the above probe is DNA encoding the active center of a protein consisting of the amino acid sequence shown in SEQ ID NO: 11 or 12.

The above Southern analysis is the same analysis as that of the above 1 and is carried out in the same manner as in the above 1, except that the above DNA is used as a probe.

The microorganisms having DNA capable of hybridization include, for example, those belonging to the genus Pseudomonas.

The microorganisms having the ability to produce a protein having 80% or more homology to the amino acid sequence shown in SEQ ID NO: 11 or 12 and having low-substrate-specific amino-acid racemase activity include microorganisms having the ability to produce a protein which consists of an amino acid sequence having at least 80% homology, preferably 90% or more homology, more preferably 95% or more homology, further preferably 98% or more homology, particularly preferably 99% or more homology to the amino acid sequence shown in SEQ ID NO: 11 or 12 as calculated by use of BLAST and FASTA described above and which has low-substrate-specific amino-acid racemase activity. Examples of the microorganisms are those belonging to the genus Pseudomonas.

The microorganisms having the ability to produce D-amino acid used in the process of the present invention also include microorganisms which are obtained by treating the above microorganisms having the ability to produce a protein having amino-acid racemase activity or the like by ordinary mutation-inducing means (A Short Course in Bacterial Genetics) such as a treatment with a mutagen (e.g., N-methyl-N'-nitro-N-nitrosoguanidine) and mutation treatment by UV irradiation or γ-rays irradiation, and then selecting microorganisms showing higher amino-acid racemase activity or the like compared with their parent strain. The ability to produce D-amino acid, for example, amino-acid racemase activity can be measured according to a conventional method.

Further, the microorganisms having the ability to produce D-amino acid used in the present invention include transformed microorganisms obtainable by introducing DNA encoding hydantoinase, D-aminoacylase, D-amino-acid amidase, D-amino-acid aminotransferase or amino-acid racemase into a host cell.

Known examples of DNAs encoding hydantoinase are DNAs described in Japanese Published Unexamined Patent Application No. 330785/02; known examples of DNAs encoding a protein having D-aminoacylase activity are DNAs described in Biosci. Biotech. Biochem., 59, 2115 (1995) and Japanese Published Unexamined Patent Application No. 275688/01; known examples of DNAs encoding a protein having D-amino-acid amidase activity are DNAs described in Applied & Environmental Microbiology, 60, 888-95 (1994); known examples of DNAs encoding a protein having D-amino-acid aminotransferase activity are DNAs described in J. Bacteriol., 185, 2418-2431 (2003) and Biochim. Biophys. Acta, 1350, 38-40 (1997); and known examples of DNAs encoding a protein having amino-acid racemase activity are DNAs derived from microorganisms belonging to the genera Escherichia [Science, 277, 1453-1474 (1997)], Bacillus [Nature, 390, 249-256 (1997)], Pseudomonas [Nature, 406, 959-964 (2000)] and Oceanobacillus [Nucleic Acids Res., 30, 3927-3935 (2002)]. Known examples of DNAs encoding a protein having low-substrate-specific amino-acid racemase activity are DNAs derived from microorganisms belonging to the genus Pseudomonas (WO03/074690). The nucleotide sequences of DNAs encoding amino-acid racemase can be obtained by searching various kinds of DNA databases using the name of the enzyme as a keyword.

Specific examples of the DNAs encoding a protein having low-substrate-specific amino-acid racemase activity preferably used in the present invention include the following [1] to [4]:
[1] DNA encoding a protein having the amino acid sequence shown in SEQ ID NO: 11 or 12;
[2] DNA having the nucleotide sequence shown in SEQ ID NO: 13 or 14;
[3] DNA which hybridizes with DNA having a nucleotide sequence complementary to the nucleotide sequence shown in SEQ ID NO: 13 or 14 under stringent conditions and which encodes a protein having amino-acid racemase activity; and
[4] DNA consisting of a nucleotide sequence which has 90% or more homology, preferably 95% or more homology, more preferably 98% or more homology, further preferably 99% or more homology to the nucleotide sequence shown in SEQ ID NO: 13 or 14 and encoding a protein having amino-acid racemase activity.

The terms "to hybridize" and "stringent conditions" as used above have the same significances as in the above 1 and the specific method for acquisition of "DNA hybridizing under stringent conditions" is also the same as in the above 1.

The above DNA capable of hybridization under stringent conditions includes, specifically, DNA having at least 90% homology, preferably 95% or more homology, more preferably 98% or more homology, further preferably 99% or more homology to the nucleotide sequence shown in SEQ ID NO: 13 or 14 as calculated by use of programs such as BLAST and FASTA described above based on the above parameters.

### 3. Preparation of DNAs Used in the Process of the Present Invention

The DNAs used in the process of the present invention, for example, DNA encoding a protein having amino-acid racemase activity and DNA encoding D-alanine-D-alanine ligase can be prepared in the following manner.

The DNA encoding a protein having amino-acid racemase activity can be obtained by Southern hybridization of a chromosomal DNA library from a microorganism, preferably one belonging to the genus Pseudomonas, using a probe designed based on a nucleotide sequence which is registered as a gene encoding amino-acid racemase in a public DNA database such as GenBank or the nucleotide sequence shown in SEQ ID NO: 13 or 14, or by PCR [PCR Protocols, Academic Press (1990)] using primer DNAs designed based on the above known gene encoding amino-acid racemase or the nucleotide sequence shown in SEQ ID NO: 13 or 14 and using, as a template, the chromosomal DNA of a microorganism, preferably one belonging to the genus Pseudomonas.

The DNA encoding D-alanine-D-alanine ligase can be obtained by Southern hybridization of a chromosomal DNA library from a microorganism, preferably one belonging to the genus Escherichia, Oceanobacillus, Synechocystis or Thermotoga, using a probe designed based on a nucleotide sequence which is registered as a gene encoding D-alanine-D-alanine ligase in a public DNA database such as GenBank or the nucleotide sequence shown in any of SEQ ID NOS: 6 to 10, or by PCR using primer DNAs designed based on the above known gene encoding D-alanine-D-alanine ligase or the nucleotide sequence shown in any of SEQ ID NOS: 6 to 10 and using, as a template, the chromosomal DNA of a microorganism, preferably one belonging to the genus Escherichia, Oceanobacillus, Synechocystis or Thermotoga.

The DNA encoding a protein having amino-acid racemase activity or the DNA encoding a protein having D-alanine-D-alanine ligase activity can also be obtained by conducting a search through various gene sequence databases for a sequence having 60% or more homology, preferably 70% or more homology, more preferably 90% or more homology, further preferably 95% or more homology, particularly preferably 98% or more homology, most preferably 99% or more homology to a known gene encoding amino-acid racemase or the nucleotide sequence shown in SEQ ID NO: 13 or 14, or a known gene encoding D-alanine-D-alanine ligase or the nucleotide sequence of DNA encoding the amino acid sequence shown in any of SEQ ID NOS: 6 to 10, and obtaining the desired DNA, based on the nucleotide sequence obtained by the search, from the chromosomal DNA or cDNA library, etc. of an organism having the nucleotide sequence according to the above-described method.

The obtained DNA, as such or after cleavage with appropriate restriction enzymes, is inserted into a vector by a conventional method, and the obtained recombinant DNA is introduced into a host cell. Then, the nucleotide sequence of the DNA can be determined by a conventional sequencing method such as the dideoxy method [Proc. Natl. Acad. Sci., USA, 74, 5463 (1977)] or by using a nucleotide sequencer such as 373A DNA Sequencer (Perkin-Elmer Corp.).

In cases where the obtained DNA is found to be a partial DNA by the analysis of nucleotide sequence, the full length DNA can be obtained by Southern hybridization of a chromosomal DNA library using the partial DNA as a probe.

It is also possible to prepare the desired DNA by chemical synthesis using a DNA synthesizer (e.g., Model 8905, PerSeptive Biosystems) based on the determined nucleotide sequence of the DNA.

Examples of the DNAs encoding a protein having amino-acid racemase activity that can be obtained by the above-described method are DNAs having the nucleotide sequences shown in SEQ ID NOS: 13 and 14, and examples of the DNAs encoding D-alanine-D-alanine ligase thus obtained are DNAs having the nucleotide sequences shown in SEQ ID NOS: 6 to 10.

### 4. Preparation of Microorganisms Obtainable by Introducing DNA Used in the Present Invention into a Host Cell

The microorganisms obtainable by introducing the DNA used in the present invention into a host cell, for example, a microorganism obtainable by introducing DNA encoding a protein having amino-acid racemase activity into a host cell and a microorganism obtainable by introducing DNA encoding a protein having D-alanine-D-alanine ligase activity into a host cell can be obtainable by introducing the DNA obtainable in the above 3 into a host cell by a conventional method. The microorganisms may have either the DNA encoding a protein having amino-acid racemase activity or the DNA encoding a protein having D-alanine-D-alanine ligase activity, or both the DNAs at the same time to be used in the process of the present invention.

On the basis of the DNA obtained in the above 3, a DNA fragment of an appropriate length comprising a region encoding a protein is prepared according to need. A microorganism having enhanced productivity of the protein can be prepared by replacing a nucleotide in the nucleotide sequence of the region encoding the protein so as to make a codon most suitable for the expression in a host cell.

The DNA fragment is inserted downstream of a promoter in an appropriate expression vector to prepare a recombinant DNA.

A transformant producing the protein of the present invention can be obtained by introducing the recombinant DNA into a host cell suited for the expression vector.

As the host cell, any microorganisms that are capable of expressing the desired gene can be used.

The expression vectors that can be employed are those capable of autonomous replication or integration into the chromosome in a microorganism and comprising a promoter at a position appropriate for the transcription of the DNA of the present invention or the DNA used in the process of the present invention.

When a procaryote such as a bacterium is used as the host cell, it is preferred that the recombinant DNA comprising the DNA encoding a protein having amino-acid racemase activity or the DNA encoding a protein having D-alanine-D-alanine ligase activity (hereinafter referred to as DNA encoding amino-acid racemase or D-alanine-D-alanine ligase) is a recombinant DNA which is capable of autonomous replication in the procaryote and which comprises a promoter, a ribosome binding sequence, the DNA of the present invention or the DNA used in the process of the present invention, and a transcription termination sequence. The recombinant DNA may further comprise a gene regulating the promoter.

Examples of suitable expression vectors are pBTrp2, pBTac1 and pBTac2 (products of Boehringer Mannheim GmbH), pHelix1 (Roche Diagnostics Corp.), pKK233-2 (Amersham Pharmacia Biotech), pSE280 (Invitrogen Corp.), pGEMEX-1 (Promega Corp.), pQE-8 (Qiagen, Inc.), pET-3 and pET-30Xa/LIC (products of Novagen, Inc.), pKYP10 (Japanese Published Unexamined Patent Application No. 110600/83), pKYP200 [Agric. Biol. Chem., 48, 669 (1984)], pLSA1 [Agric. Biol. Chem., 53, 277 (1989)], pGEL1 [Proc. Natl. Acad. Sci. USA, 82, 4306 (1985)], pBluescript II SK(+), pBluescript II KS(-) (Stratagene), pTrS30 [prepared from Escherichia coli JM109/pTrS30 (FERM BP-5407)], pTrS32 [prepared from Escherichia coli JM109/pTrS32 (FERM BP-5408)], pPAC31 (WO98/12343), pUC19 [Gene, 33, 103 (1985)], pSTV28 (Takara Bio Inc.), pUC118 (Takara Bio Inc.), pPA1 (Japanese Published Unexamined Patent Application No. 233798/88) and pJB861 [Plasmid, 38, 35-51 (1997)].

As the promoter, any promoters capable of functioning in host cells such as Escherichia coli can be used. For example, promoters derived from Escherichia coli or phage, such as trp promoter (Pₜᵣₚ), lac promoter (P_{lac}), P_{L} promoter, P_{R} promoter and P_{SE} promoter, SPO1 promoter, SPO2 promoter, penP promoter and Pm promoter can be used. Artificially designed and modified promoters such as a promoter in which two Pₜᵣₚs are combined in tandem, tac promoter, lacT7 promoter and letI promoter, etc. can also be used.

Also useful is P54-6 promoter for the expression in microorganisms belonging to the genus Corynebacterium [Appl. Microbiol. Biotechnol., 53, 674-679 (2000)].

It is preferred to use a plasmid in which the distance between the Shine-Dalgarno sequence (ribosome binding sequence) and the initiation codon is adjusted to an appropriate length (e.g., 6 to 18 nucleotides).

In the recombinant DNA wherein the DNA encoding amino-acid racemase or D-alanine-D-alanine ligase is ligated to an expression vector, the transcription termination sequence is not essential, but it is preferred to place the transcription termination sequence immediately downstream of the structural gene.

Examples of such recombinant DNAs are pJBar1 and pEDdlB described below.

Examples of procaryotes include microorganisms belonging to the genera Escherichia, Serratia, Bacillus, Brevibacterium, Corynebacterium, Microbacterium, Pseudomonas, Agrobacterium, Alicyclobacillus, Anabaena, Anacystis, Arthrobacter, Azotobacter, Chromatium, Erwinia, Methylobacterium, Phormidium, Rhodobacter, Rhodopseudomonas, Rhodospirillum, Scenedesmus, Streptmyces, Synechoccus and Zymomonas. Specific examples are Escherichia coli XL1-Blue, Escherichia coli XL2-Blue, Escherichia coli DH1, Escherichia coli DH5α, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Escherichia coli MP347, Escherichia coli NM522, Escherichia coli BL21(DE3), Bacillus subtilis ATCC 33712, Bacillus megaterium, Bacillus sp. FERM BP-6030, Brevibacterium ammoniagenes, Brevibacterium immariophilum ATCC 14068, Brevibacterium saccharolyticum ATCC 14066, Brevibacterium flavum ATCC 14067, Brevibacterium lactofermentum ATCC 13869, Corynebacterium glutamicum ATCC 13032, Corynebacterium glutamicum ATCC 14297, Corynebacterium acetoacidophilum ATCC 13870, Microbacterium ammoniaphilum ATCC 15354, Serratia ficaria, Serratia fonticola, Serratia liquefaciens, Serratia marcescens, Pseudomonas sp. D-0110, Agrobacterium radiobacter, Agrobacterium rhizogenes, Agrobacterium rubi, Anabaena cylindrica, Anabaena doliolum, Anabaena flos-aquae, Arthrobacter aurescens, Arthrobacter citreus, Arthrobacter globiformis, Arthrobacter hydrocarboglutamicus, Arthrobacter mysorens, Arthrobacter nicotianae, Arthrobacter paraffineus, Arthrobacter protophormiae, Arthrobacter roseoparaffinus, Arthrobacter sulfureus, Arthrobacter ureafaciens, Chromatium buderi, Chromatium tepidum, Chromatium vinosum, Chromatium warmingii, Chromatium fluviatile, Erwinia uredovora, Erwinia carotovora, Erwinia ananas, Erwinia herbicola, Erwinia punctata, Erwinia terreus, Methylobacterium rhodesianum, Methylobacterium extorquens, Phormidium sp. ATCC 29409, Rhodobacter capsulatus, Rhodobacter sphaeroides, Rhodopseudomonas blastica, Rhodopseudomonas marina, Rhodopseudomonas palustris, Rhodospirillum rubrum, Rhodospirillum salexigens, Rhodospirillum salinarum, Streptomyces ambofaciens, Streptomyces aureofaciens, Streptomyces aureus, Streptomyces fungicidicus, Streptomyces griseochromogenes, Streptomyces griseus, Streptomyces lividans, Streptomyces olivogriseus, Streptomyces rameus, Streptomyces tanashiensis, Streptomyces vinaceus and Zymomonas mobilis.

Introduction of the recombinant DNA can be carried out by any of the methods for introducing DNA into the above host cells, for example, the method using calcium ion [Proc. Natl. Acad. Sci. USA, 69, 2110 (1972)], the protoplast method (Japanese Published Unexamined Patent Application No. 248394/88) and electroporation [Nucleic Acids Res., 16, 6127 (1988)].

### 5. Amino Acids Used as Substrates in the Process of the Present Invention

One or more kinds of amino acids selected from the group consisting of D-amino acids and glycine which are used as substrates in the process of the present invention may be any one or more kinds of amino acids selected from the group consisting of D-amino acids and glycine, provided that the one or more kinds of amino acids are not D-alanine alone or a combination of D-alanine and D-serine. Preferred are one or more kinds of amino acids selected from the group consisting of D-alanine, D-glutamine, D-glutamic acid, glycine, D-valine, D-leucine, D-isoleucine, D-proline, D-phenylalanine, D-tryptophan, D-methionine, D-serine, D-threonine, D-cysteine, D-asparagine, D-tyrosine, D-lysine, D-arginine, D-histidine, D-aspartic acid, D-ornithine and derivatives thereof, and preferred are one or more kinds of amino acids selected from the group consisting of D-alanine, glycine, D-leucine, D-isoleucine, D-proline, D-serine, D-threonine, D-cysteine and D-lysine. The above D-amino acids may be D-amino acids obtained by any methods, for example, commercially available D-amino acids, or D-amino acids produced by chemical synthesis or by using, as an enzyme source, a culture of a microorganism having the ability to produce D-amino acid or a treated matter of the culture.

The substance converted into D-amino acid by a culture of a microorganism having the ability to produce D-amino acid or a treated matter of the culture which is used as a substrate in the present invention may be any substance that is converted into D-amino acid by a culture of such microorganism or a treated matter thereof. For example, when a microorganism having the ability to produce hydantoinase is used as the microorganism having the ability to produce D-amino acid, one or more kinds of 5-substituted-DL-hydantoin, etc. are used. When a microorganism having the ability to produce D-aminoacylase is used, one or more kinds of N-acyl-DL-amino acids, etc. are used. When a microorganism having the ability to produce D-amino-acid amidase is used, one or more kinds of DL-amino acid amides, etc. are used. When a microorganism having the ability to produce D-amino-acid aminotransferase is used, one or more kinds of α-keto acids and one or more kinds of D-amino acids, etc. are used. When a microorganism having the ability to produce amino-acid racemase is used, one or more kinds of L-α-amino acids, etc. are used.

The above L-α-amino acids may be either natural or not, or may be derivatives thereof, so far as they can be used as substrates for amino-acid racemase.

Examples of the substrates for a microorganism having the ability to produce a protein having low-substrate-specific amino-acid racemase activity include one or more kinds of amino acids comprising one or more kinds of L-α-amino acids.

As the one or more kinds of amino acids comprising one or more kinds of L-α-amino acids, any combinations of any amino acids can be used so far as they are one ore more kinds of amino acids comprising one or more kinds of L-α-amino acids. Preferred are one or two kinds of amino acids comprising one or more kinds of L-α-amino acids selected from the group consisting of L-α-amino acids and glycine.

Examples of the L-α-amino acids are L-α-alanine, L-α-glutamine, L-α-glutamic acid, L-α-valine, L-α-leucine, L-α-isoleucine, L-α-proline, L-α-phenylalanine, L-α-tryptophan, L-α-methionine, L-α-serine, L-α-threonine, L-α-cysteine, L-α-asparagine, L-α-tyrosine, L-α-lysine, L-α-arginine, L-α-histidine, L-α-aspartic acid, L-α-ornithine and derivatives thereof.

The amino acids to be used in combination with the above L-α-amino acids may be any amino acids.

Examples of the amino acids are those selected from the group consisting of L- or D-form of alanine, glutamine, glutamic acid, valine, leucine, isoleucine, proline, phenylalanine, tryptophan, methionine, serine, threonine, cysteine, asparagine, tyrosine, lysine, arginine, histidine, aspartic acid and ornithine, and glycine, and also include derivatives of these amino acids.

Examples of the derivatives of the above amino acids include hydroxyamino acids (e.g., β-hydroxyglutamine, β-hydroxyglutamic acid, γ-hydroxyglutamic acid, α-hydroxyglycine, β-hydroxyvaline, γ-hydroxyvaline, β-hydroxyleucine, γ-hydroxyleucine, δ-hydroxyleucine, β-hydroxyisoleucine, γ-hydroxyisoleucine, 3-hydroxyproline, 4-hydroxyproline, β-hydroxyphenylalanine, 3,4-dihydroxyphenylalanine, 2,4,5-trihydroxyphenylalanine, β-hydroxytryptophan, 5-hydroxytryptophan, α-hydroxymethionine, β-hydroxyserine, γ-hydroxythreonine, S-hydroxycysteine, β-hydroxyasparagine, β-hydroxytyrosine, β-hydroxylysine, γ-hydroxylysine, δ-hydroxylysine, N-hydroxylysine, β-hydroxyarginine, δ-hydroxyarginine, N-hydroxyarginine, β-hydroxyhistidine, β-hydroxyaspartic acid, β-hydroxyornithine, γ-hydroxyornithine and N-hydroxyornithine) and N-methyl amino acids (e.g., N-methyl-alanine, N-methyl-glutamine, N-methyl-glutamic acid, N-methyl-glycine, N-methyl-valine, N-methyl-leucine, N-methyl-isoleucine, N-methyl-proline, N-methylphenylalanine, N-methyl-tryptophan, N-methyl-methionine, N-methyl-serine, N-methyl-threonine, N-methyl-cysteine, N-methyl-asparagine, N-methyl-tyrosine, N-methyl-lysine, N-methyl-arginine, N-methyl-histidine, N-methyl-aspartic acid and N-methyl-ornithine).

### 6. Production Process of the Present Invention

The production process of the present invention relates to (1) a process for producing a dipeptide which comprises allowing an enzyme source, ATP and one or more kinds of amino acids selected from the group consisting of D-amino acids and glycine (provided that said one or more kinds of amino acids are not D-alanine alone or a combination of D-alanine and D-serine) to be present in an aqueous medium, said enzyme source being a culture of a microorganism having the ability to produce a protein having D-alanine-D-alanine ligase activity or a treated matter of the culture, allowing the dipeptide to form and accumulate in the aqueous medium, and recovering the dipeptide from the aqueous medium; and (2) a process for producing a dipeptide comprising D-amino acid which comprises allowing enzyme sources, ATP and a substance which is converted into the D-amino acid by a culture of a microorganism having the ability to produce the D-amino acid or a treated matter of the culture to be present in an aqueous medium, said enzyme sources being a culture of a microorganism having the ability to produce the D-amino acid or a treated matter of the culture, and a culture of a microorganism having the ability to produce a protein having D-alanine-D-alanine ligase activity or a treated matter of the culture, allowing the dipeptide comprising D-amino acid to form and accumulate in the aqueous medium, and recovering the dipeptide from the aqueous medium.

The enzyme source used in the process of the present invention can be obtained by culturing in a medium a microorganism having the ability to produce a protein having D-alanine-D-alanine ligase activity, a microorganism having the ability to produce D-amino acid, or a microorganism having both the ability to produce a protein having D-alanine-D-alanine ligase activity and the ability to produce D-amino acid.

As the medium for culturing the microorganism, any of natural media and synthetic media can be used insofar as it is a medium suitable for efficient culturing of the microorganism which contains carbon sources, nitrogen sources, inorganic salts, etc. which can be assimilated by the microorganism.

As the carbon sources, any carbon sources that can be assimilated by the microorganism can be used. Examples of suitable carbon sources include carbohydrates such as glucose, fructose, sucrose, molasses containing them, starch and starch hydrolyzate; organic acids such as acetic acid and propionic acid; and alcohols such as ethanol and propanol.

Examples of the nitrogen sources include ammonia, ammonium salts of organic or inorganic acids such as ammonium chloride, ammonium sulfate, ammonium acetate and ammonium phosphate, nitrogen-containing compounds, peptone, meat extract, yeast extract, corn steep liquor, casein hydrolyzate, soybean cake, soybean cake hydrolyzate, and various fermented microbial cells and digested products thereof.

Examples of the inorganic salts include potassium dihydrogenphosphate, dipotassium hydrogenphosphate, magnesium phosphate, magnesium sulfate, sodium chloride, ferrous sulfate, manganese sulfate, copper sulfate and calcium carbonate.

Culturing is usually carried out under aerobic conditions, for example, by shaking culture or submerged spinner culture under aeration. The culturing temperature is preferably 15 to 60°C, and the culturing period is usually 5 hours to 7 days. The pH is maintained at 4 to 10 during the culturing. The pH adjustment is carried out by using an organic or inorganic acid, an alkali solution, urea, calcium carbonate, ammonia, etc.

If necessary, antibiotics such as ampicillin and tetracycline may be added to the medium during the culturing.

When a microorganism transformed with an expression vector comprising an inducible promoter is cultured, an inducer may be added to the medium, if necessary. For example, in the case of a microorganism transformed with an expression vector comprising lac promoter, isopropyl-β-D-thiogalactopyranoside or the like may be added to the medium; in the case of a microorganism transformed with an expression vector comprising trp promoter, indoleacrylic acid or the like may be added; and in the case of a microorganism transformed with an expression vector comprising Pm promoter, m-tolic acid may be added to the medium.

Examples of the treated matters of the culture include treated matters containing living cells such as concentrated culture, dried culture, cells obtained by centrifuging the culture, products obtained by subjecting the cells to drying, freeze-drying, treatment with a surfactant, treatment with a solvent and enzymatic treatment and a product obtained by subjecting the cells to immobilization; and products obtained by subjecting the cells to ultrasonication, mechanical friction and protein fractionation, as well as enzyme preparations obtained from the cells by extraction and purification.

The culture of a microorganism having D-alanine-D-alanine ligase activity or a treated matter thereof used in the above processes (1) and (2) is used at a concentration of 1 mU/l to 1000 U/l, preferably 10 mU/l to 100 U/l, one unit (U) being defined as the activity which forms 1 mmol of dipeptide comprising D-amino acid from one or two kinds of D-amino acids at 30°C in one minute.

The culture of a microorganism having the ability to produce D-amino acid or a treated matter thereof used in the above process (2) is used at a concentration of 1 mU/l to 1000 U/l, preferably 10 mU/l to 100 U/l, one unit (U) being defined as the activity which forms 1 mmol of D-amino acid at 30°C in one minute.

One or more kinds of amino acids selected from the group consisting of D-amino acids and glycine or the substance which is converted into D-amino acid by a culture of a microorganism having the ability to produce D-amino acid or a treated matter thereof used as a substrate is used at a concentration of 1 to 500 g/l in a total amount.

The aqueous media used in the above processes (1) and (2) include water, buffers such as phosphate buffer, carbonate buffer, acetate buffer, borate buffer, citrate buffer and Tris buffer, alcohols such as methanol and ethanol, esters such as ethyl acetate, ketones such as acetone, and amides such as acetamide. The culture of a microorganism used as the enzyme source can also be used as the aqueous medium.

In the above processes (1) and (2), a surfactant or an organic solvent may be added, if necessary. Any surfactant that does not prevent the dipeptide-forming reaction can be used. Suitable surfactants include nonionic surfactants such as polyoxyethylene octadecylamine (e.g., Nymeen S-215, NOF Corporation), cationic surfactants such as cetyltrimethylammonium bromide and alkyldimethylbenzylammonium chloride (e.g., Cation F2-40E, NOF Corporation), anionic surfactants such as lauroyl sarcosinate, and tertiary amines such as alkyldimethylamine (e.g., Tertiary Amine FB, NOF Corporation), which may be used alone or in combination. The surfactant is usually used at a concentration of 0.1 to 50 g/l. As the organic solvent, xylene, toluene, aliphatic alcohols, acetone, ethyl acetate, etc. may be used usually at a concentration of 0.1 to 50 ml/l.

The reactions of the above processes (1) and (2) are carried out in the aqueous medium at pH 5 to 10, preferably pH 6 to 9, at 20 to 60°C for 1 to 96 hours.

The dipeptides produced by the above process (1) include dipeptides represented by the above formula (I) (wherein R¹ and R², which may be the same or different, each represent D-form of alanine, glutamine, glutamic acid, valine, leucine, isoleucine, proline, phenylalanine, tryptophan, methionine, serine, threonine, cysteine, asparagine, tyrosine, lysine, arginine, histidine, aspartic acid or ornithine, or glycine, preferably D-form of alanine, leucine, isoleucine, proline, serine, threonine, cysteine or lysine, or glycine; provided that both R¹ and R² cannot represent D-alanine at the same time, and when R¹ is D-alanine, R² does not represent D-serine).

The dipeptides produced by the above process (2) include dipeptides represented by the above formula (II) (wherein R³ and R⁴, which may be the same or different, each represent L- or D-form of alanine, glutamine, glutamic acid, valine, leucine, isoleucine, proline, phenylalanine, tryptophan, methionine, serine, threonine, cysteine, asparagine, tyrosine, lysine, arginine, histidine, aspartic acid or ornithine, or glycine, preferably alanine, leucine, isoleucine, proline, serine, threonine, cysteine or lysine, or glycine; provided that both R³ and R⁴ cannot represent L-form of amino acids or glycine at the same time). More preferred examples of the dipeptides produced by process (2) are dipeptides represented by the above formula (III) (wherein R⁵ and R⁶, which may be the same or different, each represent D-alanine, D-glutamine, D-glutamic acid, D-valine, D-leucine, D-isoleucine, D-proline, D-phenylalanine, D-tryptophan, D-methionine, D-serine, D-threonine, D-cysteine, D-asparagine, D-tyrosine, D-lysine, D-arginine, D-histidine, D-aspartic acid, D-ornithine or glycine, preferably D-alanine, D-leucine, D-isoleucine, D-proline, D-serine, D-threonine, D-cysteine, D-lysine or glycine; provided that both R⁵ and R⁶ cannot represent glycine at the same time).

Recovery of the dipeptide formed in the aqueous medium can be carried out by ordinary methods of isolation and purification using active carbon, ion-exchange resins, etc.

Certain embodiments of the present invention are illustrated in the following examples. These examples are not to be construed as limiting the scope of the present invention.

In the following examples, analysis and determination of dipeptides and amino acids were carried out by the following method using high performance liquid chromatography (HPLC). The dipeptides and amino acids were analyzed by HPLC after being derivatized using (2,3-dinitro-5-fluorophenyl)-L-alaninamide (Na-FDAA). Derivatization by the use of FDAA was carried out by adding 50 µl of a 0.5% solution of Na-FDAA in acetone and 40 µl of 0.5 mol/l aqueous solution of sodium carbonate to 100 µl of a sample diluted with pure water, followed by stirring, and then allowing the resulting mixture to stand at 40°C for 60 minutes.

To the above mixture were added 40 µl of 1 mol/l hydrochloric acid and 770 µl of methanol, followed by stirring. The resulting mixture was used as FDAA-derivatized sample.

The FDAA-derivatized sample was analyzed and determined using a WH-C18A column (Hitachi High-Tech Science Systems Corp., 4 x 150 mm). The following conditions were employed for HPLC analysis.

The following mobile phases were used:
mobile phase A consisting of 50 mmol/l potassium phosphate buffer (pH 2.7, adjusted with phosphoric acid), acetonitrile and methanol in 18:1:1 ratio;
mobile phase B consisting of 50 mmol/l potassium phosphate buffer (pH 2.7, adjusted with phosphoric acid), acetonitrile and methanol in 12:7:1 ratio; and
mobile phase C consisting of acetonitrile, tetrahydrofuran and methanol in 3:1:1 ratio.
The flow rate of the mobile phase was 0.5 ml/min, and the ratio of mobile phase A, mobile phase B and mobile phase C was changed as follows: minute 0 to 24, 100:0:0; minute 24 to 50, 55:45:0; minute 50 to 60, 0:100:0; minute 60 to 62, 0:0:100; and minute 62 to 80, 100:0:0. Measurement of ultraviolet absorption was carried out at 340 nm at a column temperature of 40°C.

### Example 1

### Construction of a Transformant Expressing D-Alanine-D-Alanine Ligase Gene ddlB Derived from Escherichia coli JM109

Escherichia coli JM109 was cultured in LB medium [10 g/l tryptone peptone (Difco), 10 g/l yeast extract (Difco) and 5 g/l sodium chloride] at 30°C for 24 hours, and the culture was centrifuged to obtain cells.

The chromosomal DNA of the microorganism was isolated and purified from the cells by the method described in Current Protocols in Molecular Biology.

On the basis of the nucleotide sequence of D-alanine-D-alanine ligase gene ddlB of Escherichia coli K-12 (GenBank Accession No. NC_000913), DNAs having the nucleotide sequences shown in SEQ ID NOS: 15 and 16 were synthesized using a DNA synthesizer (Model 8905, PerSeptive Biosystems, Inc.). PCR was carried out using the DNAs as a set of primers and the chromosomal DNA of Escherichia coli JM109 as a template. PCR was carried out using 50 µl of a reaction mixture comprising 0.1 µg of the chromosomal DNA, 0.5 µmol/l each of the primers, 2.5 units of ExTaq DNA polymerase (Takara Bio Inc.), 5 µl of buffer for ExTaq DNA polymerase (10 x) and 200 µmol/l each of deoxyNTPs under the following conditions:
incubation at 95°C for 3 minutes; 25 cycles of 95°C for 30 seconds, 45°C for 30 seconds and 72°C for one minute; and
a final incubation at 72°C for 5 minutes.

The resulting reaction mixture was mixed with an equal amount of phenol/chloroform (1 vol/l vol) saturated with TE [10 mmol/l Tris-HCl, 1 mmol/l EDTA (pH 8.0)].

The resulting mixture was centrifuged, and the obtained upper layer was mixed with a two-fold volume of cold ethanol and allowed to stand at -80°C for 30 minutes. The resulting solution was centrifuged, and the obtained DNA was dissolved in TE.

The DNA was digested with restriction enzymes NdeI and HindIII, and subjected to agarose gel electrophoresis to separate a ca. 0.9 kb DNA fragment. Then, the fragment was recovered from the gel and dissolved in TE. The obtained ca. 0.9 kb DNA fragment and NdeI-HindIII-digested pET21a(+) vector (Novagen, Inc.) obtained in the same manner as described above were subjected to ligation reaction using a ligation kit (Takara Bio Inc.) at 16°C for one hour.

Escherichia coli JM109 was transformed using the reaction mixture according to the above known method, spread on LB agar medium (comprising 15 g/l agar in LB medium) containing 100 µg/ml ampicillin, and cultured overnight at 30°C.

A plasmid was extracted from a colony of the transformant that grew on the medium according to the method described in Molecular Biology, Third Edition, and its structure was analyzed using restriction enzymes. It was confirmed that plasmid pEDdlB expressing the ddlB gene having the nucleotide sequence shown in SEQ ID NO: 6 was obtained. Escherichia coli JM109 carrying pEDdlB was designated as Escherichia coli JM109/pEDdlB.

### Example 2

### Construction of a Transformant Expressing D-Alanine-D-Alanine Ligase Gene ddlB Derived from Escherichia coli JM109 (2)

DNAs having the nucleotide sequences shown in SEQ ID NOS: 17 and 18 were synthesized. PCR was carried out using the DNAs as a set of primers and the chromosomal DNA of Escherichia coli JM109 prepared in Example 1 as a template. PCR was carried out using 50 µl of a reaction mixture comprising 0.1 µg of the chromosomal DNA, 0.5 µ mol/l each of the primers, 2.5 units of KOD plus DNA polymerase (Toyobo Co., Ltd.), 5 µl of buffer for KOD plus DNA polymerase (10 x) and 200 µmol/l each of deoxyNTPs under the following conditions: incubation at 94°C for 2 minutes; 25 cycles of 94°C for 30 seconds, 60°C for 30 seconds and 68°C for 90 seconds; and a final incubation at 68°C for 4 minutes.

The resulting reaction mixture was mixed with an equal amount of TE, followed by centrifugation. The obtained upper layer was mixed with a two-fold volume of cold ethanol and allowed to stand at -80°C for 30 minutes. The resulting solution was centrifuged, and the obtained DNA was dissolved in TE.

According to the protocol of Xa/LIC Cloning Kits (Novagen, Inc.), the DNA fragment amplified by PCR in the solution was inserted into pET-30 Xa/LIC vector (Novagen, Inc.).

Escherichia coli BL21(DE3) was transformed using the reaction mixture according to the above known method, spread on LB agar medium containing 50 µg/ml kanamycin, and cultured overnight at 30°C.

A plasmid was extracted from a colony of the transformant that grew on the medium according to the method described in Molecular Biology, Third Edition, and its structure was analyzed using restriction enzymes. It was confirmed that plasmid pEc-ddlB expressing the ddlB gene was obtained. Escherichia coli BL21(DE3) carrying pEc-ddlB was designated as Escherichia coli BL21(DE3)/pEc-ddlB.

### Example 3

### Construction of a Transformant Expressing D-Alanine-D-Alanine Ligase Gene ddlA Derived from Escherichia coli JM109

On the basis of the nucleotide sequence of D-alanine-D-alanine ligase gene ddlA of Escherichia coli K-12 (GenBank Accession No. NC_000913), DNAs having the nucleotide sequences shown in SEQ ID NOS: 19 and 20 were synthesized in the same manner as in Example 1. PCR was carried out using the DNAs as a set of primers and the chromosomal DNA of Escherichia coli JM109 prepared in Example 2 as a template. PCR was carried out using 50 µl of a reaction mixture comprising 0.1 µg of the chromosomal DNA, 0.5 µmol/l each of the primers, 2.5 units of KOD plus DNA polymerase (Toyobo Co., Ltd.), 5 µl of buffer for KOD plus DNA polymerase (10 x) and 200 µ mol/l each of deoxyNTPs under the following conditions:
incubation at 94°C for 2 minutes; 25 cycles of 94°C for 30 seconds, 45°C for 30 seconds and 68°C for 90 seconds; and
a final incubation at 68°C for 4 minutes.

The DNA fragment amplified by PCR was ligated to pET-30 Xa/LIC vector in the same manner as in Example 2 to construct a recombinant DNA. Then, Escherichia coli BL21(DE3) was transformed using the recombinant DNA to obtain plasmid pEc-ddlA expressing the ddlA gene having the nucleotide sequence shown in SEQ ID NO: 7. Escherichia coli BL21(DE3) carrying pEc-ddlA was designated as Escherichia coli BL21(DE3)/pEc-ddlA.

### Example 4

### Construction of a Transformant Expressing D-Alanine-D-Alanine Ligase Gene Derived from Oceanobacillus iheyensis HTE831

Oceanobacillus iheyensis HTE831 (purchased from RIKEN, Japan) was cultured in Modified Horikoshi-1 medium [10 g/l glucose, 5 g/l polypeptone (Nippon Shinyaku Co., Ltd.), 5 g/l yeast extract (Difco), 10 g/l sodium chloride, 1 g/l dipotassium hydrogenphosphate and 0.2 g/l magnesium sulfate heptahydrate, pH 9.0] at 30°C for 24 hours, and the chromosomal DNA was prepared according to a known method.

On the basis of the nucleotide sequence of D-alanine-D-alanine ligase gene ddl of Oceanobacillus iheyensis HTE831 (described in DDBJ database: http://gib.genes.nig.ac.jp/single/index.php?spid=Oihe_HTE8 31), DNAs having the nucleotide sequences shown in SEQ ID NOS: 21 and 22 were synthesized. PCR was carried out using the DNAs as a set of primers and the chromosomal DNA of Oceanobacillus iheyensis HTE831 prepared above as a template. PCR was carried out using 50 µl of a reaction mixture comprising 0.1 µg of the chromosomal DNA, 0.5 µ mol/l each of the primers, 2.5 units of KOD plus DNA polymerase, 5 µl of buffer for KOD plus DNA polymerase (10 x) and 200 µmol/l each of deoxyNTPs under the following conditions: incubation at 94°C for 2 minutes; 25 cycles of 94°C for 30 seconds, 45°C for 30 seconds and 68°C for 90 seconds; and a final incubation at 68°C for 4 minutes.

The DNA fragment amplified by PCR was ligated to pET-30 Xa/LIC vector in the same manner as in Example 3 to construct a recombinant DNA. Then, Escherichia coli BL21(DE3) was transformed using the recombinant DNA to obtain plasmid pOi-ddlA expressing the ddl gene having the nucleotide sequence shown in SEQ ID NO: 7. Escherichia coli BL21(DE3) carrying pOi-ddlA was designated as Escherichia coli BL21(DE3)/pOi-ddlA.

### Example 5

### Construction of a Transformant Expressing D-Alanine-D-Alanine Ligase Gene Derived from Synechocystis sp. PPC6803

On the basis of the nucleotide sequence of D-alanine-D-alanine ligase gene ddl of Synechocystis sp. PPC6806 (described in DNA Data Bank of Japan (DDBJ) database:
http://gib.genes.nig.ac.jp/single/index.php?spid=Syne PCC6 803), DNAs having the nucleotide sequences shown in SEQ ID NOS: 23 and 24 were synthesized in the same manner as in Example 2. PCR was carried out using the DNAs as a set of primers and the chromosomal DNA of Synechocystis sp. PPC6806 (obtained from Kazusa DNA Research Institute) as a template. PCR was carried out using 50 µl of a reaction mixture comprising 0.1 µg of the chromosomal DNA, 0.5 µ mol/l each of the primers, 2.5 units of KOD plus DNA polymerase, 5 µl of buffer for KOD plus DNA polymerase (10 x) and 200 µ mol/l each of deoxyNTPs under the following conditions: incubation at 94°C for 2 minutes; 25 cycles of 94°C for 30 seconds, 47°C for 30 seconds and 68°C for 90 seconds; and a final incubation at 68°C for 4 minutes.

The DNA fragment amplified by PCR was ligated to pET-30 Xa/LIC vector in the same manner as in Example 2 to construct a recombinant DNA. Then, Escherichia coli BL21(DE3) was transformed using the recombinant DNA to obtain plasmid pSs-ddlA expressing the ddlA gene having the nucleotide sequence shown in SEQ ID NO: 9. Escherichia coli BL21(DE3) carrying pSs-ddlA was designated as Escherichia coli BL21 (DE3) /pSs-ddlA.

### Example 6

### Construction of a Transformant Expressing a Low-Substrate-Specific Amino-Acid Racemase Gene Derived from Pseudomonas putida IFO12996

Pseudomonas putida IFO12996 was cultured in an ordinary bouillon medium (Kyokuto Pharmaceutical Industrial Co., Ltd.) at 30°C for 24 hours, and the culture was centrifuged to obtain cells.

The chromosomal DNA of the microorganism was isolated and purified from the cells by the method described in Current Protocols in Molecular Biology.

On the basis of the nucleotide sequence of a low-substrate-specific amino-acid racemase gene of Pseudomonas putida (WO03/074690), DNAs having the nucleotide sequences shown in SEQ ID NOS: 25 and 26 were synthesized using a DNA synthesizer (Model 8905, PerSeptive Biosystems, Inc.). PCR was carried out using the DNAs as a set of primers and the chromosomal DNA of Pseudomonas putida IFO12996 as a template. PCR was carried out using 50 µl of a reaction mixture comprising 0.1 µg of the chromosomal DNA, 0.5 µ mol/l each of the primers, 2.5 units of ExTaq DNA polymerase, 5 µl of buffer for ExTaq DNA polymerase (10 x) and 200 µmol/l each of deoxyNTPs under the following conditions: incubation at 95°C for 3 minutes; 25 cycles of 95°C for 90 seconds, 60°C for 60 seconds and 72°C for 90 seconds; and a final incubation at 72°C for 4 minutes.

The resulting reaction mixture was mixed with an equal amount of phenol/chloroform saturated with TE, followed by centrifugation. The obtained upper layer was mixed with a two-fold volume of cold ethanol and allowed to stand at -80°C for 30 minutes. The resulting solution was centrifuged, and the obtained DNA was dissolved in 20 µl of TE.

The obtained DNA fragment was digested with restriction enzymes BamHI and EcoRI, and subjected to agarose gel electrophoresis to separate a ca. 1.2 kb DNA fragment. The fragment was recovered from the gel and dissolved in TE. Similarly, pSTV28 vector (Takara Bio Inc.) digested with BamHI and EcoRI was prepared. The ca. 1.2 kb DNA fragment and pSTV28 vector digested with the restriction enzymes were subjected to ligation reaction using a ligation kit at 16°C for one hour.

Escherichia coli JM109 was transformed using the reaction mixture according to the above known method, spread on LB agar medium containing 25 µg/ml chloramphenicol, and cultured overnight at 30°C.

A plasmid was extracted from a colony of the transformant that grew on the medium according to the method described in Molecular Biology, Third Edition, and its structure was analyzed using restriction enzymes. It was confirmed that plasmid pSTBAR having DNA represented by the nucleotide sequence shown in SEQ ID NO: 13 was obtained.

On the basis of the nucleotide sequence of the low-substrate-specific amino-acid racemase gene of Pseudomonas putida (WO03/074690), DNAs having the nucleotide sequences shown in SEQ ID NOS: 27 and 28 were synthesized. PCR was carried out using the DNAs as a set of primers and the above-obtained pSTBAR as a template. PCR was carried out using 50 µl of a reaction mixture comprising 0.1 µg of the plasmid DNA, 0.5 µmol/l each of the primers, 2.5 units of ExTaq DNA polymerase, 5 µl of buffer for ExTaq DNA polymerase (10 x) and 200 µmol/l each of deoxyNTPs under the following conditions: incubation at 95°C for 3 minutes; 25 cycles of 95°C for 30 seconds, 58°C for 30 seconds and 72°C for 90 seconds; and a final incubation at 72°C for 4 minutes.

The resulting reaction mixture was mixed with an equal amount of phenol/chloroform saturated with TE, followed by centrifugation. The obtained upper layer was mixed with a two-fold volume of cold ethanol and allowed to stand at -80°C for 30 minutes. The resulting solution was centrifuged, and the obtained DNA was dissolved in 20 µl of TE.

The obtained DNA fragment was partially digested with restriction enzymes AflIII and EcoRI, and subjected to agarose gel electrophoresis. A ca. 1.2 kb DNA fragment was recovered from the gel and dissolved in TE. The solution (5 µl) and pJB861 vector [Plasmid, 38, 35-51 (1997)] digested with AflIII and EcoRI were subjected to ligation reaction using a ligation kit at 16°C for one hour.

Escherichia coli JM109 was transformed using the reaction mixture according to the above known method, spread on LB agar medium containing 50 µg/ml kanamycin, and cultured overnight at 30°C.

A plasmid was extracted from a colony of the transformant that grew on the medium according to the method described in Molecular Biology, Third Edition, and its structure was analyzed using restriction enzymes. It was confirmed that plasmid pJBar1 expressing the low-substrate-specific amino-acid racemase gene having the nucleotide sequence shown in SEQ ID NO: 13 was obtained. Escherichia coli JM109 carrying pJBar1 was designated as Escherichia coli JM109/pJBar1.

### Example 7

### Preparation of D-Alanine-D-Alanine Ligase

Escherichia coli BL21(DE3) was transformed using the plasmid pEDdlB prepared in Example 1 according to the above known method, spread on LB agar medium containing 100 µg/ml ampicillin, and cultured overnight at 30°C.

Escherichia coli BL21(DE3) carrying pEDdlB was obtained from a colony of the transformant which grew and was designated as Escherichia coli BL21(DE3)/pEDdlB.

Escherichia coli BL21(DE3)/pEDdlB was cultured in LB medium containing 100 µg/ml ampicillin at 37°C. When OD (660 nm) reached 0.1, IPTG was added to the culture to give a concentration of 0.1 mmol/l, followed by further culturing for 2 hours. The resulting culture was centrifuged and the obtained cells were suspended in a 0.1 mol/l Tris-HCl buffer (pH 8.0). The suspension was subjected to ultrasonication and centrifugation to obtain the supernatant as a crude enzyme solution containing D-alanine-D-alanine ligase.

### Example 8

### Production of D-Alanyl-D-Alanine Using D-Alanine as a Substrate

A reaction mixture (0.4 ml) comprising 0.1 mol/l Tris-HCl buffer (pH 7.5) containing the crude enzyme solution containing D-alanine-D-alanine ligase prepared in Example 7 (protein amount: 0.4 mg/ml), 12.5 mmol/l D-alanine, 10 mmol/l ATP, 10 mmol/l magnesium chloride and 10 mmol/l potassium chloride was prepared and subjected to reaction at 37°C for 5 hours. The reaction mixture was analyzed by HPLC, whereby 4.7 mmol/l D-alanyl-D-alanine was detected in the reaction mixture.

### Example 9

### Production of D-Seryl-D-Serine Using D-Serine as a Substrate

A reaction mixture (0.4 ml) comprising 0.1 mol/l Tris-HCl buffer (pH 7.5) containing the crude enzyme solution containing D-alanine-D-alanine ligase prepared in Example 7 (protein amount: 0.4 mg/ml), 12.5 mmol/l D-serine, 10 mmol/l ATP, 10 mmol/l magnesium chloride and 10 mmol/l potassium chloride was prepared and subjected to reaction at 37°C for 5 hours. The reaction mixture was analyzed by HPLC, whereby 4 mmol/l D-seryl-D-serine was detected in the reaction mixture.

### Example 10

### Production of Glycyl-Glycine Using D-Alanine-D-Alanine Ligase

A reaction mixture (0.1 ml) comprising 0.1 mol/l Tris-HCl buffer (pH 7.5) containing the crude enzyme solution containing D-alanine-D-alanine ligase prepared in Example 7 (protein amount: 0.5 mg/ml), 40 mmol/l glycine, 20 mmol/l ATP, 20 mmol/l magnesium chloride and 10 mmol/l potassium chloride was prepared and subjected to reaction at 37°C for 24 hours. The reaction mixture was analyzed by HPLC, whereby 4 mmol/l glycyl-glycine was detected in the reaction mixture.

### Example 11

### Production of D-Cysteinyl-D-Cysteine Using D-Cysteine as a Substrate

A reaction mixture (0.1 ml) comprising 0.1 mol/l Tris-HCl buffer (pH 7.5) containing the crude enzyme solution containing D-alanine-D-alanine ligase prepared in Example 7 (protein amount: 0.5 mg/ml), 40 mmol/l D-cysteine, 20 mmol/l ATP, 20 mmol/l magnesium chloride and 10 mmol/l potassium chloride was prepared and subjected to reaction at 37°C for 24 hours. The reaction mixture was analyzed by HPLC, whereby 3 mmol/l D-cysteinyl-D-cysteine was detected in the reaction mixture.

### Example 12

### Production of D-Threonyl-D-Threonine Using D-Threonine as a Substrate

A reaction mixture (0.1 ml) comprising 0.1 mol/l Tris-HCl buffer (pH 7.5) containing the crude enzyme solution containing D-alanine-D-alanine ligase prepared in Example 7 (protein amount: 0.5 mg/ml), 40 mmol/l D-threonine, 20 mmol/l ATP, 20 mmol/l magnesium chloride and 10 mmol/l potassium chloride was prepared and subjected to reaction at 37°C for 24 hours. The reaction mixture was analyzed by HPLC, whereby 1 mmol/l D-threonyl-D-threonine was detected in the reaction mixture.

### Example 13

### Preparation of D-Alanine-D-Alanine Ligase and Low-Substrate-Specific Amino-Acid Racemase

Escherichia coli BL21(DE3)/pEDdlB prepared in Example 6 was transformed using the plasmid pJBar1 prepared in Example 6 according to the above known method, spread on LB agar medium containing 100 µg/ml ampicillin and 50 µg/ml kanamycin, and cultured overnight at 30°C.

A transformant carrying two plasmid DNAs (pEDdlB and pJBar1) was obtained from a colony of the transformant that grew on the medium and was designated as Escherichia coli BL21(DE3)/pJBar1,pEDdlB.

Escherichia coli BL21(DE3)/pJBar1,pEDdlB was cultured in LB medium containing 100 µg/ml ampicillin and 50 µg/ml kanamycin at 37°C. When OD (660 nm) reached 0.1, IPTG and m-tolic acid were added to the culture to give concentrations of 0.1 mmol/l and 3 mmol/l, respectively, followed by further culturing for 2 hours. The resulting culture was centrifuged and the obtained cells were suspended in a 0.1 mol/l Tris-HCl buffer (pH 8.0). The suspension was subjected to ultrasonication and centrifugation to obtain the supernatant as a crude enzyme solution containing D-alanine-D-alanine ligase and amino-acid racemase.

### Example 14

### Production of D-Alanyl-D-Alanine Using L-Alanine as a Substrate

A reaction mixture (0.4 ml) comprising 0.1 mol/l Tris-HCl buffer (pH 7.5) containing the crude enzyme solution containing D-alanine-D-alanine ligase and amino-acid racemase prepared in Example 13 (protein amount: 0.4 mg/ml), 20 mmol/l L-alanine, 10 mmol/l ATP, 10 mmol/l magnesium chloride and 10 mmol/l potassium chloride was prepared and subjected to reaction at 37°C for 3 hours. The reaction mixture was analyzed by HPLC, whereby 6.8 mmol/l D-alanyl-D-alanine was detected in the reaction mixture.

### Example 15

### Production of D-Seryl-D-Serine Using L-Serine as a Substrate

A reaction mixture (0.4 ml) comprising 0.1 mol/l Tris-HCl buffer (pH 7.5) containing the crude enzyme solution containing D-alanine-D-alanine ligase and amino-acid racemase prepared in Example 13 (protein amount: 0.4 mg/ml), 20 mmol/l L-serine, 10 mmol/l ATP, 10 mmol/l magnesium chloride and 10 mmol/l potassium chloride was prepared and subjected to reaction at 37°C for 3 hours. The reaction mixture was analyzed by HPLC, whereby 3 mmol/l D-seryl-D-serine was detected in the reaction mixture.

### Example 16

### Production of D-Alanyl-D-Serine Using L-Alanine and L-Serine as Substrates

A reaction mixture (0.4 ml) comprising 0.1 mol/l Tris-HCl buffer (pH 7.5) containing the crude enzyme solution containing D-alanine-D-alanine ligase and amino-acid racemase prepared in Example 13 (protein amount: 0.4 mg/ml), 10 mmol/l L-alanine, 10 mmol/l L-serine, 10 mmol/l ATP, 10 mmol/l magnesium chloride and 10 mmol/l potassium chloride was prepared and subjected to reaction at 37°C for 3 hours. The reaction mixture was analyzed by HPLC, whereby 4.5 mmol/l D-alanyl-D-serine and 2 mmol/l D-alanyl-D-alanine were detected in the reaction mixture.

### Example 17

### Construction of a Transformant Expressing a D-Alanine-D-Alanine Ligase Gene Derived from Thermotoga maritima ATCC 43589

On the basis of the nucleotide sequence of a D-alanine-D-alanine ligase gene of Thermotoga maritima ATCC 43589 [described in DNA Data Bank of Japan (DDBJ) database:
http://gib.genes.nig.ac.jp/common/info.php?spid=Tmar_MSB8& ftid=267], DNAs having the nucleotide sequences shown in SEQ ID NOS: 29 and 30 were synthesized in the same manner as in Example 2. PCR was carried out using the DNAs as a set of primers and the chromosomal DNA of Thermotoga maritima ATCC 43589 as a template. PCR was carried out using 50 µl of a reaction mixture comprising 0.1 µg of the chromosomal DNA, 0.5 µmol/l each of the primers, 2.5 units of KOD plus DNA polymerase, 5 µl of buffer for KOD plus DNA polymerase (10 x) and 200 µmol/l each of deoxyNTPs under the following conditions: incubation at 94°C for 2 minutes; 25 cycles of 94°C for 30 seconds, 47°C for 30 seconds and 68°C for 90 seconds; and a final incubation at 68°C for 4 minutes.

The DNA fragment amplified by PCR was ligated to pET-30 Xa/LIC vector in the same manner as in Example 2 to construct a recombinant DNA. Then, Escherichia coli BL21(DE3) was transformed using the recombinant DNA to obtain plasmid pTmDdl expressing the D-alanine-D-alanine ligase gene having the nucleotide sequence shown in SEQ ID NO: 10. Escherichia coli BL21(DE3) carrying pTmDdl was designated as Escherichia coli BL21(DE3)/pTmDdl.

### Example 18

### Production of D-Amino Acid-Comprising Dipeptides Using D-Alanine-D-Alanine Ligase of Thermotoga maritima ATCC 43589

A crude enzyme solution containing D-alanine-D-alanine ligase derived from Thermotoga maritima ATCC 43589 was prepared from Escherichia coli BL21(DE3)/pTmDdl in the same manner as in Example 7. A reaction mixture (0.1 ml) comprising 0.1 mol/l Tris-HCl buffer (pH 7.5) containing the crude enzyme solution (protein amount: 0.5 mg/ml), 40 mmol/l D-alanine, 20 mmol/l ATP, 20 mmol/l magnesium chloride and 10 mmol/l potassium chloride was prepared and subjected to reaction at 37°C for 5 hours. The reaction mixture was analyzed by HPLC, whereby 11 mmol/l D-alanyl-D-alanine was detected in the reaction mixture. When D-serine was used in place of D-alanine in the above reaction, 9 mmol/l D-seryl-D-serine was detected; when D-cysteine was used, 9 mmol/l D-cysteinyl-D-cysteine was detected; when D-threonine was used, 4 mmol/l D-threonyl-D-threonine was detected; and when glycine was used, 8 mmol/l glycyl-glycine was detected.

### Industrial Applicability

The present invention provides an efficient process for producing various kinds of dipeptides comprising D-amino acids.

### SEQUENCE LISTING FREE TEXT

SEQ ID NO: 15 - Description of Artificial Sequence: Synthetic DNA
SEQ ID NO: 16 - Description of Artificial Sequence: Synthetic DNA
SEQ ID NO: 17 - Description of Artificial Sequence: Synthetic DNA
SEQ ID NO: 18 - Description of Artificial Sequence: Synthetic DNA
SEQ ID NO: 19 - Description of Artificial Sequence: Synthetic DNA
SEQ ID NO: 20 - Description of Artificial Sequence: Synthetic DNA
SEQ ID NO: 21 - Description of Artificial Sequence: Synthetic DNA
SEQ ID NO: 22 - Description of Artificial Sequence: Synthetic DNA
SEQ ID NO: 23 - Description of Artificial Sequence: Synthetic DNA
SEQ ID NO: 24 - Description of Artificial Sequence: Synthetic DNA
SEQ ID NO: 25 - Description of Artificial Sequence: Synthetic DNA
SEQ ID NO: 26 - Description of Artificial Sequence: Synthetic DNA
SEQ ID NO: 27 - Description of Artificial Sequence: Synthetic DNA
SEQ ID NO: 28 - Description of Artificial Sequence: Synthetic DNA
SEQ ID NO: 29 - Description of Artificial Sequence: Synthetic DNA
SEQ ID NO: 30 - Description of Artificial Sequence: Synthetic DNA

## Claims

1. A process for producing a dipeptide, which comprises: allowing an enzyme source, ATP and one or more kinds of amino acids selected from the group consisting of D-amino acids and glycine (provided that said one or more kinds of amino acids are not D-alanine alone or a combination of D-alanine and D-serine) to be present in an aqueous medium, said enzyme source being a culture of a microorganism having the ability to produce a protein having D-alanine-D-alanine ligase activity or a treated matter of the culture; allowing the dipeptide to form and accumulate in the aqueous medium; and recovering the dipeptide from the aqueous medium.

2. The process according to Claim 1, wherein the D-amino acid is produced by using a culture of a microorganism having the ability to produce the D-amino acid or a treated matter of the culture as an enzyme source.

3. A process for producing a dipeptide comprising D-amino acid which comprises: allowing enzyme sources, ATP and a substance which is converted into the D-amino acid by a culture of a microorganism having the ability to produce the D-amino acid or a treated matter of the culture to be present in an aqueous medium, said enzyme sources being a culture of a microorganism having the ability to produce the D-amino acid or a treated matter of the culture, and a culture of a microorganism having the ability to produce a protein having D-alanine-D-alanine ligase activity or a treated matter of the culture; allowing the dipeptide comprising D-amino acid to form and accumulate in the aqueous medium; and recovering the dipeptide from the aqueous medium.

4. The process according to any one of Claims 1 to 3, wherein the protein having D-alanine-D-alanine ligase activity is a protein selected from the group consisting of the following [1] to [3]:
[1] a protein having the amino acid sequence shown in any of SEQ ID NOS: 1 to 5;
[2] a protein consisting of an amino acid sequence wherein one or more amino acid residues are deleted, substituted or added in the amino acid sequence shown in any of SEQ ID NOS: 1 to 5 and having D-alanine-D-alanine ligase activity; and
[3] a protein consisting of an amino acid sequence which has 80% or more homology to the amino acid sequence shown in any of SEQ ID NOS: 1 to 5 and having D-alanine-D-alanine ligase activity.

5. The process according to any one of Claims 1 to 3, wherein the microorganism having the ability to produce a protein having D-alanine-D-alanine ligase activity is a microorganism obtainable by introducing DNA encoding the protein having D-alanine-D-alanine ligase activity into a host cell.

6. The process according to Claim 5, wherein the DNA encoding a protein having D-alanine-D-alanine ligase activity is DNA selected from the group consisting of the following [1] to [4]:
[1] DNA encoding a protein having the amino acid sequence shown in any of SEQ ID NOS: 1 to 5;
[2] DNA having the nucleotide sequence shown in any of SEQ ID NOS: 6 to 10;
[3] DNA which hybridizes with DNA having a nucleotide sequence complementary to the nucleotide sequence shown in any of SEQ ID NOS: 6 to 10 under stringent conditions and which encodes a protein having D-alanine-D-alanine ligase activity; and
[4] DNA consisting of a nucleotide sequence which has 90% or more homology to the nucleotide sequence shown in any of SEQ ID NOS: 6 to 10 and encoding a protein having D-alanine-D-alanine ligase activity.

7. The process according to Claim 2 or 3, wherein the microorganism having the ability to produce D-amino acid is a microorganism having the ability to produce a protein having the activity of hydantoinase, D-aminoacylase, D-amino-acid amidase, D-amino-acid transaminase or amino-acid racemase.

8. The process according to Claim 7, wherein the protein having amino-acid racemase activity is a protein having low-substrate-specific amino-acid racemase activity.

9. The process according to Claim 8, wherein the protein having low-substrate-specific amino-acid racemase activity is a protein selected from the group consisting of the following [1] to [3]:
[1] a protein having the amino acid sequence shown in SEQ ID NO: 11 or 12;
[2] a protein consisting of an amino acid sequence wherein one or more amino acid residues are deleted, substituted or added in the amino acid sequence shown in SEQ ID NO: 11 or 12 and having low-substrate-specific amino-acid racemase activity; and
[3] a protein consisting of an amino acid sequence which has 80% or more homology to the amino acid sequence shown in SEQ ID NO: 11 or 12 and having low-substrate-specific amino-acid racemase activity.

10. The process according to Claims 2 and 3, wherein the microorganism having the ability to produce D-amino acid is a microorganism obtainable by introducing DNA encoding a protein having the activity of hydantoinase, D-aminoacylase, D-amino-acid amidase, D-amino-acid aminotransferase or amino-acid racemase into a host cell.

11. The process according to Claim 10, wherein the DNA encoding a protein having amino-acid racemase activity is DNA encoding low-substrate-specific amino-acid racemase.

12. The process according to Claim 11, wherein the DNA encoding a protein having low-substrate-specific amino-acid racemase activity is DNA selected from the group consisting of the following [1] to [4]:
[1] DNA encoding a protein having the amino acid sequence shown in SEQ ID NO: 11 or 12;
[2] DNA having the nucleotide sequence shown in SEQ ID NO: 13 or 14;
[3] DNA which hybridizes with DNA having a nucleotide sequence complementary to the nucleotide sequence shown in SEQ ID NO: 13 or 14 under stringent conditions and which encodes a protein having low-substrate-specific amino-acid racemase activity; and
[4] DNA consisting of a nucleotide sequence which has 90% or more homology to the nucleotide sequence shown in SEQ ID NO: 13 or 14 and encoding a protein having low-substrate-specific amino-acid racemase activity.

13. The process according to any one of Claims 1, 2 and 4 to 6, wherein the one or more kinds of amino acids selected from the group consisting of D-amino acids and glycine are D-amino acids selected from the group consisting of D-alanine, D-glutamine, D-glutamic acid, glycine, D-valine, D-leucine, D-isoleucine, D-proline, D-phenylalanine, D-tryptophan, D-methionine, D-serine, D-threonine, D-cysteine, D-asparagine, D-tyrosine, D-lysine, D-arginine, D-histidine, D-aspartic acid and D-ornithine (provided that said one or more kinds of amino acids are not D-alanine alone or a combination of D-alanine and D-serine).

14. The process according to any one of Claims 3 to 12, wherein the substance which is converted into D-amino acid by a culture of a microorganism having the ability to produce the D-amino acid or a treated matter of the culture is one or more kinds of 5-substituted-DL-hydantoin, one or more kinds of N-acyl-DL-amino acids, one or more kinds of DL-amino acid amides, one or more kinds of α-keto acids and one or more kinds of D-amino acids, or one or more kinds of amino acids comprising one or more kinds of L-α-amino acids.

15. The process according to Claim 14, wherein the one or more kinds of amino acids comprising one or more kinds of L-α-amino acids comprise one or more kinds of amino acids selected from the group consisting of L- or D-form of alanine, glutamine, glutamic acid, valine, leucine, isoleucine, proline, phenylalanine, tryptophan, methionine, serine, threonine, cysteine, asparagine, tyrosine, lysine, arginine, histidine, aspartic acid and ornithine, and glycine.

16. The process according to any one of Claims 1, 2 and 4 to 12, wherein the dipeptide is a dipeptide represented by formula (I):
R¹ - R² (I)
(wherein R¹ and R², which may be the same or different, each represent D-form of alanine, glutamine, glutamic acid, valine, leucine, isoleucine, proline, phenylalanine, tryptophan, methionine, serine, threonine, cysteine, asparagine, tyrosine, lysine, arginine, histidine, aspartic acid or ornithine, or glycine; provided that both R¹ and R² cannot represent D-alanine at the same time, and when R¹ is D-alanine, R² does not represent D-serine).

17. The process according to any one of Claims 3 to 12, wherein the dipeptide comprising D-amino acid is a dipeptide represented by formula (II):
R³ - R⁴ (II)
(wherein R³ and R⁴, which may be the same or different, each represent L- or D-form of alanine, glutamine, glutamic acid, valine, leucine, isoleucine, proline, phenylalanine, tryptophan, methionine, serine, threonine, cysteine, asparagine, tyrosine, lysine, arginine, histidine, aspartic acid or ornithine, or glycine; provided that both R³ and R⁴ cannot represent L-form of amino acids or glycine at the same time).

18. The process according to any one of Claims 3 to 12, wherein the dipeptide comprising D-amino acid is a dipeptide represented by formula (III):
R⁵ - R⁶ (III)
(wherein R⁵ and R⁶, which may be the same or different, each represent D-alanine, D-glutamine, D-glutamic acid, D-valine, D-leucine, D-isoleucine, D-proline, D-phenylalanine, D-tryptophan, D-methionine, D-serine, D-threonine, D-cysteine, D-asparagine, D-tyrosine, D-lysine, D-arginine, D-histidine, D-aspartic acid, D-ornithine or glycine; provided that both R⁵ and R⁶ cannot represent glycine at the same time).
